(19) Europäisches Patentamt
European Patent Office
Office européen des brevets

(11) **EP 4 113 155 A1**

(12) **EUROPÄISCHE PATENTANMELDUNG**

(43) Veröffentlichungstag:
04.01.2023 Patentblatt 2023/01

(21) Anmeldenummer: 22173971.7

(22) Anmeldetag: **18.05.2022**

(51) Internationale Patentklassifikation (IPC):
$G01S\ 7/02^{(2006.01)}$ $\qquad$ $G01S\ 7/40^{(2006.01)}$
$G01S\ 13/00^{(2006.01)}$ $\qquad$ $G01S\ 13/87^{(2006.01)}$
$G01S\ 13/88^{(2006.01)}$ $\qquad$ $G01N\ 22/04^{(2006.01)}$
$G01N\ 33/24^{(2006.01)}$ $\qquad$ $G01S\ 7/41^{(2006.01)}$
$G01V\ 3/12^{(2006.01)}$

(52) Gemeinsame Patentklassifikation (CPC):
G01S 13/003; G01N 22/04; G01N 33/246;
G01S 7/027; G01S 7/40; G01S 7/411; G01S 13/87;
G01S 13/885; G01V 3/12

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **30.06.2021 DE 102021116885**

(71) Anmelder: **Forschungszentrum Jülich GmbH 52425 Jülich (DE)**

(72) Erfinder: **Steinbeck, Leon 52062 Aachen (DE)**

(74) Vertreter: **Paul & Albrecht Patentanwälte PartG mbB Stresemannallee 4b 41460 Neuss (DE)**

(54) **VERFAHREN ZUR ZERSTÖRUNGSFREIEN UNTERSUCHUNG EINER PROBE MIT EINER RADAR-, INSBESONDERE GEO-RADAR-EINRICHTUNG, RADAR-EINRICHTUNG, COMPUTERPROGRAMM UND COMPUTERPROGRAMMPRODUKT**

(57) Die vorliegende Erfindung betrifft ein Verfahren zur zerstörungsfreien Untersuchung einer Probe (7) mit einer Radar-Einrichtung (1), wobei die Radar-Einrichtung (1) mehrere Transceiver (2) umfasst, bei dem
- eine oder mehrere reziproke Messungen zwischen Transceiver-Paaren durchgeführt werden (Schritt S1), wobei eine reziproke Messung jeweils eine Hin- und eine Rück-Messung einschließt,
- unter Rückgriff auf die oder die jeweilige Ankunftszeit des Messsignals an dem einen Transceiver (2) während der oder der jeweiligen Hin-Messung, Hin-Ankunftszeit ($t_{a\,i\,j}$), und der Ankunftszeit des Messsignals an dem anderen Transceiver während der oder der jeweiligen Rück-Messung, Rück-Ankunftszeit ($t_{a\,j\,i}$), wenigstens eine Laufzeit ($t_p$, $t_{p\,i\,j}$) des Messsignales zwischen den beiden Transceiver (2) ermittelt wird (Schritt S2), und
- aus der wenigstens einen Laufzeit ($t_p$, $t_{pij}$, $t_{pji}$) auf Eigenschaften der zu untersuchenden Probe (7) geschlossen wird (Schritt S3).

FIG. 1

EP 4 113 155 A1

**Beschreibung**

Die vorliegende Erfindung betrifft ein Verfahren zur zerstörungsfreien Untersuchung einer Probe mit einer Radar-, insbesondere Geo-Radar-Einrichtung. Darüber hinaus betrifft die Erfindung eine Radar-, insbesondere Geo-Radar-Einrichtung, ein Computerprogramm und ein Computerprogrammprodukt.

Radar ist die Abkürzung für RAdio Detection And Ranging bzw. RAdio Direction And Ranging und wird auch als Oberbegriff für eine Mehrzahl von Ortungsverfahren und zugehörigen Einrichtungen, die elektromagnetische Wellen (EM) im Radiofrequenzbereich nutzen, verwendet. Als Radiowellen sind dabei insbesondere solche zu verstehen, deren Frequenz(en) unterhalb von 3000 GHz liegt (liegen).

Es ist bekannt, Radarmessungen zur Untersuchung des Bodens durchzuführen. In diesem Falle spricht man auch von Boden- oder Geo-Radar bzw. englisch: Ground Penetrating Radar, kurz GPR. Das Boden- bzw. Geo-Radar ist eine nicht-invasive elektromagnetische (EM) geophysikalische Technik zur Untersuchung des oberflächennahen Untergrunds von Böden durch Aussenden von hochfrequenten EM-Wellen in den Boden und Empfang eines Antwortsignals über Antennen (Jol 2008). GPR liefert eine höhere räumliche Auflösung als die elektromagnetische Induktion (z. B. Doolittle et al. 2014) und hat eine schnellere Datenerfassung als elektrische Widerstandsmessungen (z. B. Samouëlian et al. 2005). Die Ausbreitung der emittierten EM-Wellen hängt von der relativen dielektrischen Permittivität $\varepsilon_r$ und der elektrischen Leitfähigkeit $\sigma$ des Untergrundes ab. Typische Werte der Permittivität liegen im Bereich von $\varepsilon_r \approx 1$ für Luft und $\varepsilon_r \approx 80$ für Wasser, wodurch die Permittivität hochsensitiv auf den Wassergehalt des Bodens (englisch: Soil Water Content, kurz SWC) reagiert (Topp et al. 1980; Dobson et al. 1985; Roth et al. 1990). Dies erlaubt die Messung und Korrelation von SWC-Variationen unter Nutzung geeigneter petrophysikalischer Modelle (Huisman et al. 2003; Paz et al. 2017; Klotzsche, Jonard, et al. 2018; Wu et al. 2019). Die Messung des SWC und damit von Strömungs- und Transportprozessen mit hoher Genauigkeit unterstützt die Landwirtschaft (Bolten et al. 2010; De Benedetto et al. 2019; Yang et al. 2021), die Flurneuordnung (X. Xu et al. 2014) und Leckerkennung (Hyun et al. 2007). Generell gilt, dass ein besseres Verständnis von Bodenparametern hilft, ausgereifte Boden-Pflanze-Atmosphäre-Modelle zu entwickeln (Vereecken et al. 2016). Neben Anwendungen im Zusammenhang mit dem SWC wird GPR auch u.a. für die nicht-invasive Inspektion von Straßen (Saarenketo et al. 2000; Hammond et al. 2011; Ihamouten et al. 2018), Betonbrücken (Hugenschmidt et al. 2006; Dinh et al. 2019), Eisenbahnen (Ciampoli et al. 2019), die Bewertung von kontaminierten Bereichen (Catapano et al. 2014; Wijewardana et al. 2017) und die Detektion von Landminen (Sai et al. 2004; Lameri et al. 2017) eingesetzt.

Eine besondere GPR-Messkonfiguration ist der sogenannte Crosshole-Aufbau, bei dem Sende- und Empfangsantennen innerhalb von Bohrlöchern platziert werden (Slob et al. 2010; Qin et al. 2021). Der Crosshole-Aufbau in Kombination mit der Erfassung von Signalen über viele variierende Antennenpositionen (englisch: Multi-Offset-Gathern) ermöglicht es dem GPR-Nutzer, ein Tomographiebild von $\varepsilon_r$ und $\sigma$ und damit den SWC zwischen Bohrlöchern mit einer höheren räumlichen Auflösung in tieferen Bodenbereichen im Vergleich zu On- oder Off-Ground-Messungen zu bestimmen (Binley et al. 2001; Liu et al. 2017). Die Analyse der bohrlochübergreifenden GPR-Daten mit einer Volle-Wellenform-Inversion (englisch: Full-Waveform-Inversion, kurz FWI, Ernst et al. 2007; Meles et al. 2010; Klotzsche, Vereecken, et al. 2019), bei der das komplette Signal zur Inversion verwendet wird, liefert im Vergleich zu Standardansätzen räumlich höher aufgelöste Bilder der Permittivitäts- und Leitfähigkeitsverteilung im Dezimeterbereich (Gueting et al. 2017; Zhou et al. 2020).

Im Allgemeinen leiten GPR-Systeme die Permittivität des Bodens von der Bestimmung der Laufzeit und damit die Geschwindigkeit der EM-Wellen im Boden ab. Um die Signallaufzeit zwischen Sende- (Tx) und Empfangsantenne (Rx) zu bestimmen, muss eine Zeitreferenz für die gemessenen Signale definiert werden, die als zeitlicher Nullpunkt t0 bezeichnet wird (Cassidy 2009).

Die Korrektur des zeitlichen Nullpunktes (englisch: time-zero correction) ist ein wesentlicher Schritt insbesondere bei der Datenvorverarbeitung von Bodenradarmessungen, um eine genaue Signallaufzeit zwischen Sende- und Empfangsantennen zu erhalten. Die Korrektur des zeitlichen Nullpunktes zielt darauf ab, eine zuverlässige Zeitreferenz zu finden und stellt sicher, dass keine zusätzliche Signallaufzeit aus den Messgeräten, z. B. Systemtiming und Kabellänge, in den Daten enthalten ist (Olhoeft 2000). Darüber hinaus kann die Korrektur des zeitlichen Nullpunktes Änderungen des zeitlichen Nullpunktes durch z. B. thermische Drifts, beschädigte Kabel oder elektronische Instabilität (Huisman et al. 2003) überwachen und ist daher zeitabhängig. Des Weiteren weisen Klotzsche, Vereecken, et al. 2019 darauf hin, dass die genaue Korrektur des zeitlichen Nullpunktes ein entscheidender Schritt in der Vorverarbeitung (englisch: pre-processing) ist, um die FWI korrekt auf die Daten anzuwenden.

Der zeitliche Nullpunkt ist üblicherweise definiert als

$$t_0 := t_a - \frac{d}{c_0} \qquad (1)$$

wobei $t_a$- die Ankunftszeit der direkten Luftwelle zwischen *Tx* und *Rx* bei *Rx*, d die Ausbreitungsdistanz der direkten Luftwelle und co die Lichtgeschwindigkeit ist (Annan 2003). Das $t_0$ entspricht mit anderen Worten der Summe der Laufzeiten, die über den Luftweg zwischen der *Tx*- und der *Rx*-Antenne hinausgehen, nachdem die Datenaufnahme beim Empfänger gestartet wurde.

Es ist zu beachten, dass die Luftwelle unter einer Sichtlinienbedingung immer zuerst bei *Rx* ankommt, wobei der kürzeste Weg zwischen *Tx* und *Rx* durch Luft verläuft. Aufgrund von Antennen-Nahfeld-Effekten und einer Interferenz der Luft- und Bodenwelle kann die direkte Luftwelle eine Geschwindigkeit haben, die von dem angenommenen co abweicht (Gerhards et al. 2008), was zu einem falschen zeitlichen Nullpunkt bei Verwendung von (1) führt. Daher ist es vorteilhaft, eine lineare Regression zu nutzen, um den zeitlichen Nullpunkt und die Signalgeschwindigkeit *v* aus mehreren Messungen mit unterschiedlichen Abständen *d* zwischen *Tx* und *Rx* abzuleiten, wobei

$$t_a(d) = t_0 + \frac{d}{v} \qquad (2)$$

Die für die Anwendung von (2) erforderlichen Kalibrierdaten werden entweder durch Änderung des Abstands zwischen *Tx* und *Rx* für mehrere Messungen oder durch sequentielles Anheben von *Tx* und *Rx* über einen bekannten Reflektor und Messung der Zwei-Wege-Laufzeit erfasst (Yelf 2004). Anstatt den zeitlichen Nullpunkt direkt über (1) oder (2) zu messen, besteht ein anderer Ansatz darin, den zeitlichen Nullpunkt über eine Inversion zu bestimmen. Gerhards et al. 2008 implementierten eine Mehrpunkt-Inversion, die eine Zielfunktion, die die Luft-Wellenausbreitungszeit als freien Parameter enthält, minimiert, um ein mehrkanaliges GPR-System zu kalibrieren. Kaufmann et al. 2020 stellten kürzlich eine neuartige Zeitkalibrierungsmethode vor, um das WARR-Gerät, ein GPR-Bodensystem, das eine Sendeantenne und sieben Empfangsantennen nutzt, zu kalibrieren (Diamanti et al. 2018). Jede Empfangsantenne hat ihre eigene Datenerfassungs-Hardware, was eine Korrektur des zeitlichen Nullpunktes für jede Empfangsantenne erforderlich macht. Das finale Kalibrierungsschema liefert einen präzisen zeitlichen Nullpunkt , indem es das Minimum einer Zielfunktion findet, während die Kalibrierungsmessung selbst mit Messzeiten von etwa 40 Minuten zeitaufwendig ist. In dieser Studie wurde beobachtet, dass, obwohl der Aufbau genau gleich war, die Differenz der zeitlichen Nullpunkte jedes Tx-Rx-Paares zum ersten Paar stabil war und nur eine gesamte Verschiebung der zeitlichen Nullpunkte bestimmt werden muss. Radzevicius 2015 verwendete einen kombinierten Kleinste-Quadrateund Grid-Such-Algorithmus, um den zeitlichen Nullpunkt zusammen mit weiteren Modellparametern zu bestimmen. Viriyametanont et al. 2008 führten eine effektive Korrektur des zeitlichen Nullpunkesbasierend auf der direkten Wellenausbreitung in Beton anstelle der Nutzung der direkten Luftwelle durch. Diese Methode erfordert bekannte Reflektortiefen innerhalb des Betons.

Bei Bohrloch-übergreifenden Messungen ist die direkte Luftwelle zwischen *Tx* und *Rx*, die in getrennten Bohrlöchern angeordnet sind, nicht messbar. Daher werden die Antennen nach mehreren Crosshole-Aufnahmen, z. B. nach 10 Messungen, wiederholt in der Luft positioniert (Oberröhrmann et al. 2012), um Korrekturmessungen des zeitlichen Nullpunktes gemäß (2) durchzuführen und die zeitliche Variabilität des zeitlichen Nullpunktes zu erfassen. Anschließend werden die zeitlichen Nullpunkte aus aufeinanderfolgenden Korrekturmessungen interpoliert, um einen individuellen zeitlichen Nullpunkt für jede Bohrlochmessung zu erhalten. Oberröhrmann et al. 2012 führten eine Kreuzkorrelationsmethode ein, um die Genauigkeit des zeitlichen Nullpunktes für Bohrlochmessungen durch die Verknüpfung von Signalen, die an vergleichbaren Tx-Rx-Positionen erfasst wurden, zu verbessern. Wie von Klotzsche, Vereecken, et al. 2019 hervorgehoben, ist dies aufgrund der Empfindlichkeit der FWI gegenüber kleinen Fehlern des zeitlichen Nullpunktes besonders wichtig.

Bisher wurde die Crosshole-GPR-FWI hauptsächlich zur Untersuchung von Grundwasserleitern und damit verbundenen Prozessen auf Dezimeterskala verwendet. In den letzten Jahren ist die Notwendigkeit, kleinere Strukturen zu detektieren und detailliertere und schnellere Prozesse, wie bevorzugte Fließwege oder Wurzelzonen, zu kartieren, immer wichtiger geworden, um Modellvorhersagen zu verbessern. Um dies zu erreichen, sind höherfrequente Antennen, die eine höhere Auflösung und schnellere Messtechniken bieten, unerlässlich.

Bei fest installierten Radar-Einrichtungen ist die Verwendung von vorbekannten Ansätzen, welche die Luftwelle für eine genaue Zeit-Null-Korrektur nutzen, nicht möglich. Dies insbesondere, da die Option einer vorübergehenden Positionierung in Luft für die Kalibrierung nicht zur Verfügung steht. Vor allem unbekannte Materialeigenschaften zwischen einem beliebigen Paar von Sendeund Empfangsantennen stehen einer konventionellen Korrektur des zeitlichen Nullpunktes entgegen.

Es ist daher eine Aufgabe der vorliegenden Erfindung, ein Verfahren zur zerstörungsfreien Untersuchung einer Probe mit einer Radar-, insbesondere Geo-Radar-Einrichtung, anzugeben, welches auch für fest installierte Einrichtungen eine Kalibrierung ermöglich und zuverlässige Ergebnisse liefert.

Diese Aufgabe wird gelöst durch ein Verfahren zur zerstörungsfreien Untersuchung einer Probe mit einer Radar-, insbesondere Geo-Radar-Einrichtung, wobei die Radar-Einrichtung mehrere Transceiver, die jeweils sowohl als Sender als auch als Empfänger für elektromagnetische Messsignale fungieren können, umfasst, bei dem

- eine oder mehrere reziproke Messungen zwischen Transceiver-Paaren durchgeführt werden, wobei eine reziproke Messung jeweils eine Hinund eine Rück-Messung einschließt, wobei für die Hin-Messung der eine Transceiver des oder des jeweiligen Paares als Sender und der andere Transceiver als Empfänger fungiert, und für die Rück-Messung der andere Transceiver des oder des jeweiligen Paares als Sender und der eine Transceiver als Empfänger fungiert,
- unter Rückgriff auf die oder die jeweilige Ankunftszeit des Messsignals an dem einen Transceiver, insbesondere einer Antenne dieses, während der oder der jeweiligen Hin-Messung, Hin-Ankunftszeit, und der Ankunftszeit des Messsignals an dem anderen Transceiver, insbesondere einer Antenne dieses, während der oder der jeweiligen Rück-Messung, Rück-Ankunftszeit, wenigstens eine Laufzeit des Messsignales zwischen den beiden Transceivern ermittelt wird, und
- aus der wenigstens einen Laufzeit auf Eigenschaften der zu untersuchenden Probe, insbesondere eine Permittivität dieser, geschlossen wird.

Die vorliegende Erfindung basiert mit anderen Worten auf dem Grundgedanken, alternativ zu einer Kalibrierung in Luft reziproke Messungen zwischen jeweils zwei Transceivern, also Transceiver-Paaren, durchzuführen und die Ergebnisse dieser, insbesondere die zugehörigen Ankunftszeiten beider Messrichtungen zu nutzen, um die Laufzeit des Messsignals zwischen den beiden Transceivern zu bestimmen. Von dieser können dann in an sich bekannter Weise Eigenschaften der Probe, beispielsweise deren Permittivität, abgeleitet werden.

Das Messsignal kann sich während der reziproken Messung(en) durch die zu untersuchende Probe, deren Eigenschaften, etwa Permittivität noch nicht bekannt sind, ausbreiten und von dem jeweils als Empfänger dienenden Transceiver aufgenommen werden, nachdem es diese zumindest abschnittsweise durchdrungen hat. Es ist nicht erforderlich, durch ein Kalibriermedium mit bekannten Eigenschaften, insbesondere bekannter Permittivität, wie etwa Luft, zu messen, um den zeitlichen Nullpunkt zu ermitteln. Durch die erfindungsgemäß vorgesehene(n) reziproke Messung(en) kann auf eine solche Kalibriermessung verzichtet werden, so dass das erfindungsgemäße Verfahren insbesondere auch mit fest installierten Radar-Einrichtungen durchgeführt werden kann und zuverlässige Ergebnisse liefert.

Unter einem Transceiver ist insbesondere in an sich bekannter Weise eine Einrichtung bzw. ein Modul zu verstehen, die eine Kombination aus Sender und Empfänger darstellt und entsprechend ausgebildet und/oder eingerichtet ist. Bevorzugt umfasst jeder Transceiver einen Sende-Schaltkreis und einen Empfangs-Schaltkreis sowie wenigstens eine, insbesondere genau eine Antenne. Ist genau eine Antenne vorhanden, dient diese sowohl dem Senden als auch dem Empfangen von elektromagnetischen Messsignalen. Jeder Transceiver umfasst weiterhin zweckmäßiger Weise einen Signalgenerator für die Signalerzeugung. Besonders bevorzugt gilt, dass alle Transceiver der Radar-Einrichtung baugleich ausgebildet sind.

Bei der Hin-Messung fungiert der eine Transceiver des bzw. des jeweiligen Transceiver-Paares als Sender und emittiert zweckmäßiger Weise ein elektromagnetisches Messsignal in Richtung des anderen Transceivers. Der andere Transceiver des Paares fungiert dann als Empfänger, der das Messsignal erfasst. Für die Rück-Messung sind Sender- und Empfänger-Rolle dann vertauscht. Dies kann man auch als wechselseitige (reziproke) Messung bezeichnen. Wenn vorliegend von einer reziproken Messung gesprochen wird, ist die Kombination aus einer solchen Hin- und Rück-Messung zwischen zwei betrachteten Transceivern, also einem Transceiver-Paar, gemeint.

Es kann sein, dass genau eine Laufzeit ermittelt wird, insbesondere aus der Hin- und Rück-Ankunftszeit genau einer reziproken Messung. Auch ist es möglich, dass mehrere Laufzeiten ermittelt werden, insbesondere für den Fall, dass mehrere reziproke Messungen durchgeführt werden. In diesem Falle gilt bevorzugt, dass aus der Hin- und Rückankunftszeit jeweils einer reziproken Messung jeweils eine Laufzeit ermittelt wird. Werden mehrere reziproken Messungen durchgeführt, wird bevorzugt für alle reziproken Messungen jeweils eine Laufzeit zwischen den beiden jeweiligen Transceivern ermittelt. Bevorzugt wird angenommen, dass die Laufzeit für die Hin- und die Rück-Messung gleich ist, bzw. Abweichungen vernachlässigbar sind, insbesondere weniger als 5 ps betragen, und die eine aus den beiden Ankunftszeiten einer reziproken Messung ermittelte Laufzeit als Laufzeit für beide Richtungen angenommen wird.

Dass unter Rückgriff auf eine Hin- und Rück-Ankunftszeit einer reziproken Messung eine Laufzeit zwischen einem Transceiver-Paar ermittelt wird, bedeutet insbesondere bzw. schließt insbesondere mit ein, dass wenigstens eine Formel verwendet wird, in welche (u.a.) die Hin- und Rückankunftszeit als Input einfließen und welche als Ergebnis die Laufzeit liefert.

Bei der oder den Laufzeiten, die ermittelt werden, handelt es sich insbesondere um die Laufzeit(en) zwischen den beiden Antennen des bzw. des jeweiligen Transceiver-Paares, für das die (jeweilige) reziproke Messung durchgeführt wird. In analoger Weise gilt bevorzugt, dass als Hin-Ankunftszeit die Ankunftszeit an der Antenne des (jeweiligen) Transceiver bei der (jeweiligen) Hin-Messung und als Rück-Ankunftszeit die Ankunftszeit an der Antenne des (jeweiligen) Transceiver bei der (jeweiligen) Rück-Messung erfasst wird.

Die wenigstens eine Laufzeit kann auf die erfindungsgemäße Weise ermittelt werden, ohne dass es einer Bestimmung eines zeitlichen Nullpunktes über eine Kalibriermessung in Luft bedarf. Aufgrund der Nutzung der Hin- und Rück-Ankunftszeit einer oder mehrerer reziproker Messungen ist die Kalibriermessung in Luft nicht mehr nötig. Dies ist zum Beispiel bei fest installierten Radar-Einrichtungen, bei denen eine (Um-)Positionierung von Transceivern für eine Messung in Luft nicht ohne weiteres möglich ist, ganz besonders vorteilhaft.

Die Hin- und Rück-Ankunftszeit bzw. -Ankunftszeiten können prinzipiell auf beliebige Weise erfasst, insbesondere messtechnisch ermittelt werden. Als Messsignale können insbesondere Peak-, beispielsweise Gauss-förmige Signale dienen, die von dem (jeweils) als Sender dienenden Transceiver gesendet und von dem (jeweils) als Empfänger dienenden Transceiver empfangen werden. Für die Ankunftszeit eines Peak-, etwa Gauss-förmigen Messsignals kann prinzipiell auf einen beliebigen Punkt auf dem (Gauss-förmigen) Peak abgestellt werden, etwa auf einen Punkt vor, an bzw. auf der ansteigenden Flanke, wie die Halbwertsbreite (FWHM), oder auch das Maximum etc. Zweckmäßiger Weise wird dabei, zumindest was die Hin- und Rück-Ankunftszeit einer reziproken Messung angeht, jeweils auf den gleichen Punkt abgestellt. Natürlich kann im Falle mehrerer auch für alle reziproken Messungen auf den gleichen Punkt abgestellt werden.

Die vorliegende Erfindung geht von folgenden Überlegungen aus.

Für n Transceiver markiert $t = (t_1, ..., t_n)^T$ die Triggerzeiten aller Transceiver.

Für eine komplette Tomographie-Messung wird zweckmäßiger Weise jeder Transceiver einmal zum Senden verwendet, während die anderen n-1 Transceiver als Empfänger dienen. Unbekannte Proben-, beispielweise Bodeneigenschaften und damit Signalgeschwindigkeiten zwischen beliebigen Antennenpaaren verhindern zunächst die Verwendung von direkten Signalen wie in (1) für eine Korrektur des zeitlichen Nullpunktes.

Als nächstes werden wichtige Zeitsegmente für eine Messung zwischen zwei Transceivern *i* und *j* erörtert.

Die Zeitachse beginnt mit der Aussendung des Triggers, der beispielsweise von einem entsprechenden Modul, insbesondere Trigger-Modul der Radar-Einrichtung, ausgegeben werden kann. Bevorzugt ein Signalgenerator des sendenden Transceivers i beginnt zum Zeitpunkt $t_i$, während die Datenerfassung des Empfangs-Transceivers j zum Zeitpunkt $t_j$ beginnt. $t_i$ und $t_j$ sind die Trigger-Zeiten der Transceiver i und j. $t_{Tx}$ und $t_{Rx}$ sind die analogen Schaltkreis-Laufzeiten (z.B. innerhalb von Kabeln). Diese beschreiben die Zeiten, die ein Puls braucht, um in dem *Tx* bzw. Rx Schaltkreis zu propagieren, diesen mit anderen Worten zu durchlaufen.

Das Signal wird von der Antenne von Transceiver i zum Zeitpunkt $t_i + t_{Tx}$ in das Lysimeter emittiert. Unter Verwendung der Definition von $t_0 = t_a - t_p$, wobei $t_p$ die Laufzeit des Signals zwischen den Antennen ist, erhält man

$$t_{0\ i\ j} = t_i - t_j + t_{\mathrm{Tx}} + t_{\mathrm{Rx}} = t_{i\ j} + t_{\mathrm{Tx}} + t_{\mathrm{Rx}}\ , \qquad (3)$$

wobei

$$t_{i\ j} := t_i - t_j \qquad (4)$$

als das Trigger-Offset der Transceiver *i* und *j* definiert wird. Daher setzt die Bestimmung des zeitlichen Nullpunktes für ein beliebiges Transceiver-Paar die Kenntnis der Differenz zwischen den Triggerzeiten der entsprechenden Transceiver voraus. Die Triggerzeiten unterscheiden sich vor allem durch unterschiedliche Kabellängen zu den Transceivern. Insbesondere für den Fall, dass $t_{Tx}$ und $t_{Rx}$ für alle Transceiver gleich, bzw. Unterschiede vernachlässigbar, insbesondere < 5 ps, und stabil sind, müssen diese Zeiten nur einmal kalibriert werden. Für baugleiche Transceiver gilt ferner, dass sie im Vergleich zu den Unterschieden in den Triggerzeiten vernachlässigbare Unterschiede, insbesondere von weniger als 5 ps, in $t_{Tx}$ und $t_{Rx}$ haben und als gleich angenommen werden können.

Im Rahmen der vorliegenden Erfindung kann insbesondere eine paarweise Kalibrierung zum Einsatz kommen. Die paarweise Kalibrierung zielt auf die Bestimmung der Laufzeit eines Signals zwischen zwei beliebigen Antennen ab. Die paarweise Kalibrierung basiert auf der Möglichkeit, jeden Transceiver entweder als *Tx* oder *Rx* zu verwenden. Dies erlaubt die Durchführung der reziproken, mit anderen Worten wechselseitigen Messungen zwischen zwei beliebigen Transceivern *i* und *j,* also beliebigen Transceiver-Paaren, was zu folgenden Ergebnissen führt

$$\mathrm{Tx}_i, \mathrm{Rx}_j: t_i + t_{\mathrm{Tx}} + t_{\mathrm{p}\ i\ j} + t_{\mathrm{Rx}} - t_j = t_{\mathrm{a}\ i\ j} \qquad (5)$$

$$\mathrm{Tx}_j, \mathrm{Rx}_i: t_j + t_{\mathrm{Tx}} + t_{\mathrm{p}\ j\ i} + t_{\mathrm{Rx}} - t_i = t_{\mathrm{a}\ j\ i} \qquad (6)$$

ta i j beschreibt die Ankunftszeit des Signals am Empfänger, konkret der Empfänger-Antenne, wenn Transceiver i sendet und j empfängt, $t_{a\,ij}$ die Ankunftszeit für den umgekehrten Fall. $t_{p\,ij}$ ist die Laufzeit des Signals von der sendenden Antenne von Transceiver i zu der empfangenden Antenne von Transcei*ver j*, $t_{p\,ij}$ wiederum für den umgekehrten Fall.

Der Begriff reziproke Messung bezieht sich, wie angemerkt, immer auf ein Paar von zwei normalen Messungen, insbesondere wie in (5) und (6) (Hin-Messung und Rück-Messung). Es ist zweckmäßig anzunehmen, dass die Laufzeit $t_p$ unabhängig von der Richtung ist, insbesondere aufgrund der Tatsache, dass es sich bei der Probe um ein passives und somit lineares Element handelt und folglich $t_p := t_{p\,i\,j} = tp_{j\,i}$ gilt. Addiert man (5) und (6) und ordnet um, so erhält man

$$t_p = \frac{t_{a\,i\,j} + t_{a\,j\,i}}{2} - (t_{\mathrm{Tx}} + t_{\mathrm{Rx}}) \qquad (7)$$

Eine besonders bevorzugte Ausführungsform des erfindungsgemäßen Verfahrens zeichnet sich entsprechend dadurch aus, dass die Laufzeit zwischen einem Transceiver $i$ und einem Transceiver $j$ eines Transceiver-Paares bestimmt wird mittels der Formel (7), wobei $t_{a\,i\,j}$ die Hin-Ankunftszeit, $t_{a\,j\,i}$ die Rück-Ankunftszeit, $t_{\mathrm{Tx}}$ die analoge Sendepfad-Laufzeit und $t_{\mathrm{Rx}}$ die analoge Empfangspfad-Laufzeit des jeweiligen Transceivers ist. Zweckmäßiger Weise wird dabei davon ausgegangen, dass die Summe von $t_{\mathrm{Tx}}$ und $t_{\mathrm{Rx}}$ bekannt ist.

Dies verdeutlicht, dass die unbekannte Laufzeit $t_p$ des Signals zwischen i und j durch das unbekannte Medium, die normalerweise nur über einen kalibrierten zeitlichen Nullpunkt bestimmt werden kann, erfindungsgemäß direkt durch eine reziproke Messung ermittelt werden kann. Dies insbesondere, wenn Signallaufzeit innerhalb der Analogschaltung $t_{Tx} + t_{Rx}$, bekannt ist. Der Vorteil dieser Methode ist, dass jedes einzelne Transceiver-Paar unabhängig von seiner Position kalibriert werden kann und dass $t_{Tx} + t_{Rx}$ nur einmal kalibriert werden muss, dies unter der Annahme, dass es sich um einen zeitlich konstanten Wert für alle Transceiver-Paare handelt.

Bei Bodenuntersuchungen beispielsweise kann es sein, dass die Reziprozität für Messungen durch die Probe unter bestimmten Bodenbedingungen verletzt ist, wie z. B. beim Vorhandensein von Wellenleitern mit niedriger Geschwindigkeit, z. B. einem Neigungswinkel oder diskontinuierlichen Schichten (Klotzsche, van der Kruk, et al. 2014). Daher werden die reziproken Messungen in bevorzugter Ausgestaltung verglichen und nur dann zur Kalibrierung verwendet, wenn die Signalformen gleich sind. Um schnelle Bodenveränderungen sichtbar zu machen, sind schnellstmögliche Tomographiemessungen zweckmäßig. In diesen Fällen kann die paarweise Kalibrierung den Nachteil haben, dass jede Signalspur zweimal in die Messdaten aufgenommen werden muss, um reziproke Messungen zu erhalten. Wenn statische Bodenbedingungen während der gesamten Messung angenommen werden, ist die reziproke Spur redundant und erhöht die Messzeit für ein vollständiges Tomogramm. Andererseits können dynamische Bewässerungsprozesse die Zeitinvarianz des Bodens stören ($t_{p\,i\,j} \neq t_{p\,j\,i}$) und könnten bei Verwendung von (7) womöglich zu nicht vollständig korrekten Ergebnissen führen.

Die paarweise Kalibrierung ist besonders gut geeignet für passive lineare zeitinvariante Systeme, die ein reziprokes Verhalten zeigen. In diesen Szenarien ist die paarweise Kalibrierung die besonders bevorzugte Methode. Wenn jedoch die Reziprozität für ein oder mehrere Transceiver-Paare nicht gegeben ist, oder wenn schnelle Messungen erforderlich sind, kann in vorteilhafter Weiterbildung die Kalibrierung erweitert bzw. durch den im Folgenden beschriebenen Mesh-Kalibrierungsansatz ergänzte oder auch ersetzt werden.

Die in bevorzugter Weiterbildung des erfindungsgemäßen Verfahrens vorgesehene Mesh-Kalibrierung (deutsch: Gitter-Kalibrierung) zielt auf die Bestimmung von $t_p$ über einmalig ermittelte zeitliche Nullpunkte ab und basiert auf einer Überlagerung von reziproken Messungen insbesondere zwischen benachbarten Transceivern. Dadurch kann der Einfluss der Probe, etwa des nahen Bodens, auf das Signal in der Radar-Einrichtung minimiert werden. Die reziproken Messungen sorgen dafür, dass $t_{Tx}$, $t_{RX}$ und $t_p$ aus den folgenden Gleichungen eliminiert werden. Der Trigger-Offset $t_{ij}$ zwischen zwei beliebigen Transceivern wird gemäß dieser bevorzugten Ausführungsform des erfindungsgemäßen Verfahrens über eine Überlagerung der Koppelsignale zwischen bevorzugt benachbarten Transceivern berechnet. Um dies zu veranschaulichen, wird zunächst (6) von (5) subtrahiert, was

$$t_{i\,j} = \frac{t_{a\,i\,j} - t_{a\,j\,i}}{2} \qquad (8)$$

liefert, so dass man durch Subtraktion der bekannten Hin- und Rück-Ankunftszeiten den gesuchten Wert in (3) erhält. Gleichung (8) gilt auch für benachbarte Transceiver. Wenn z. B. $t_{1\,3}$, mit anderen Worten das Trigger-Offset von einem Transceiver-Paar welches einen ersten und dritten Transceiver von einer Gruppe von Transceivern, die beispielweise in einer Reihe nebeneinander angeordnet sind, bestimmt werden soll, so wird bevorzugt zunächst eine reziproke Messung zwischen den Transceivern 1 und 2 durchgeführt, wobei Transceiver 2 ein direkter Nachbar von Transceiver 1 ist. Aus (8) folgt, dass

$$t_{1\,2} = t_1 - t_2 = \frac{t_{a\,1\,2} - t_{a\,2\,1}}{2} \qquad (9)$$

ist. Wenn man auf die gleiche Weise mit einer reziproken Messung zwischen den Transceivern 2 und 3 weitermacht, die wiederum bevorzugt direkte Nachbarn in der Reihe sind, erhält man

$$t_{2\ 3} = t_2 - t_3 = \frac{t_{a\ 2\ 3} - t_{a\ 3\ 2}}{2} \qquad (10)$$

Die Berechnung von (9) + (10) liefert

$$t_{1\ 3} = t_{1\ 2} + t_{2\ 3} = t_1 - t_2 + t_2 - t_3 = t_1 - t_3 \qquad (11)$$

Dieser Vorgang kann wiederholt werden, bis jede Verzögerungszeit berechnet ist. Es ist möglich, dass jeder zusätzliche Transceiver, der zur Bestimmung des Trigger-Offsets für beliebige zwei Transceiver verwendet wird, zu einer Fehlerverstärkung durch ungenaue Messungen führen kann.

Ein anspruchsvollerer Ansatz besteht darin, eine Matrix A so zu definieren, dass

$$A\ t = t_{\mathrm{measured}}, \qquad (12)$$

wobei t die Triggerzeiten aller vorhandenen Transceiver sind, und $t_{measured}$ ein Vektor, der die gemessenen Trigger-Offsets benachbarter Transceiver, wie in (9) und (10), enthält.

Für

$$B\ t = t_{\mathrm{searched}}, \qquad (13)$$

wobei B die gesuchten Transceiver-Kombinationen definiert und $t_{searched}$ der Vektor ist, der die gesuchten Trigger-Offsets enthält (z. B. $t_{1\ 3}$), folgt, dass

$$t_{\mathrm{searched}} = B\ A^+ t_{\mathrm{measured}} \qquad (14).$$

A+ bezeichnet die Pseudoinverse von A, die durch eine QR-Faktorisierung oder Singulär-Wert-Dekomposition berechnet werden kann. Unter Verwendung der Matrizen A und B wird es also möglich, jedes Trigger-Offset einfach auf Basis von Kopplungsmessungen zu bestimmen. Diese Vorgehensweise hat Gemeinsamkeiten mit derjenigen von Xu et. Al 1993, die über eine Matrizenmultiplikation Widerstandsdaten synthetisieren. Schließlich wird die Ausbreitungszeit eines Signals zwischen beliebigen Transceivern $i$ und $j$ bestimmt als

$$t_{\mathrm{p}\ i\ j} = t_{a\ i\ j} - t_{i\ j} - (t_{\mathrm{Tx}} + t_{\mathrm{Rx}}) \qquad (15).$$

Im Lichte des Vorstehenden gilt, dass sich eine weitere besonders bevorzugte Ausführungsform des erfindungsgemäßen Verfahrens dadurch auszeichnet, dass die Laufzeit $t_{\mathrm{p}\ i\ j}$ zwischen einem Transceiver i und einem Transceiver $j$ eines Transceiver-Paares bestimmt wird mittels der Formel (15), wobei $t_{a\ i\ j}$ die Hin-Ankunftszeit, $t_{\mathrm{Tx}}$ die analoge Laufzeit des Sende-Schaltkreises und $t_{\mathrm{Rx}}$ die analoge Laufzeit Empfangs-Schaltkreises des jeweiligen Transceivers ist. Es wird insbesondere davon ausgegangen, dass $t_{\mathrm{Tx}}$ und $t_{\mathrm{Rx}}$ bekannt sind. Bevorzugt wird ferner angenommen, dass $t_{\mathrm{Tx}}$ und $t_{\mathrm{Rx}}$ und für alle Transceiver gleich sind bzw. sich nur vernachlässigbar unterscheiden, insbesondere um weniger als 5 ps.

$t_{i\ j}$ ist das Trigger-Offset der Transceiver i und j eines Transceiver-Paares, das definiert ist durch Gleichung (4), mit der Trigger-Zeit $t_i$ des Transceivers $i$ und der Trigger-Zeit $t_j$ des Transceivers $j$.

Besonders bevorzugt gilt, dass das Trigger-Offsets berechnet werden mittels der vorstehenden Formel (8).

Weiter bevorzugt gilt, dass die Trigger-Offsets für Paare von Transceivern, die keine unmittelbaren Nachbarn sind, aus den Trigger-Offsets von insbesondere dazwischenliegenden, jeweils in vertikaler und/oder horizontaler und/oder diagonaler Richtung direkt benachbarten Transceiver-Paaren berechnet werden. Man kann auch von Nachbar-Kopplungsmessungen sprechen.

Wie vorstehend dargelegt, hat sich im Rahmen des Mesh-Kalibrierungsansatzes die Verwendung von Matrizen als besonders geeignet erwiesen. In Weiterbildung des erfindungsgemäßen Verfahrens ist daher vorgesehen, dass Trigger-Offsets $t_{i\ j}$ von Transceiver-Paaren als Input für die Formel (15) unter Verwendung zweier Matrizen ermittelt werden. Dann gilt bevorzugt, dass eine Matrix A definiert wird, so dass

$$A \; t = t_{\mathrm{measured}}$$

gilt, wobei t die Trigger-Zeiten mehrerer, insbesondere aller Transceiver umfasst, und $t_{\mathrm{measured}}$ ein Vektor ist, der die Trigger-Offset von Transceiver-Paaren, bevorzugt solchen, die in vertikaler und/oder horizontaler und/oder diagonaler Richtung direkt benachbart sind, umfasst, die über $t_{i\;j} = \frac{t_{\mathrm{a}\;i\;j} - t_{\mathrm{a}\;j\;i}}{2}$ ermittelt werden, und

$$B \; t = t_{\mathrm{searched}}$$

gilt, wobei die weitere Matrix $B$ gesuchte Transceiver-Kombinationen umfasst, und der Vektor $t_{\mathrm{searched}}$ gesuchte Trigger-Offsets enthält, und

$$t_{\mathrm{searched}} = B \; A^{+} t_{\mathrm{measured}}$$

gilt, wobei $A^{+}$ die Pseudoinverse der Matrix A ist, die bevorzugt durch QR-Faktorisierung oder Singulärwert-Zerlegung berechnet wird.

Es ist zu beachten, dass die Verwendung von Koppelsignalen, insbesondere Nachbar-Koppelsignalen, für die Kalibrierung keine zusätzlichen Messungen während einer Tomographie erfordert, da jeder Transceiver einmal als *Tx* für eine komplette Tomographie verwendet wird. Der einzige zusätzliche Aufwand besteht darin, insbesondere benachbarte Transceiver eines Tx-Transceivers als Rx-Transceiver zu verwenden.

Die Mesh-Kalibrierung kann beispielsweise vor Bewässerungsprozessen genutzt werden, um eine Korrektur des zeitlichen Nullpunktes basierend auf Gleichung (3) für beliebige zwei Transceiver zu erzielen. Wenn der Boden dann schnelle Änderungen erfährt, ist es ausreichend, wenn die Hälfte aller Transceiver als *Tx* für die Bodenmessung verwendet werden und demgemäß kann die Aufnahmezeit reduziert werden. Im Gegensatz zu dem paarweisen Kalibrier-Verfahren müssen bei der die Mesh-Kalibrierung nur einmal Kalibrierdaten bestimmt werden.

Es kann vorgesehen sein, dass die Transceiver in einer oder mehreren Reihen und/oder einer oder mehreren Spalten angeordnet sind.

Weiterhin kann vorgesehen sein, dass reziproke Messungen für Transceiver-Paare durchgeführt werden, die in horizontaler Richtung direkt zueinander benachbart sind. Alternativ oder zusätzlich kann vorgesehen sein, dass reziproke Messungen für Transceiver-Paare durchgeführt werden, die in vertikaler Richtung direkt zueinander benachbart sind. Auch ist es möglich, dass reziproke Messungen für Transceiver-Paare durchgeführt werden, die in diagonaler Richtung direkt zueinander benachbart sind.

Reziproke Messungen zwischen Nachbar-Transceivern werden insbesondere für die Mesh-Kalibrierung durchgeführt.

Sind Transceiver in einer oder mehreren Reihen und/oder in einer oder mehreren Spalten angeordnet, erfolgen bevorzugt für alle jeweils benachbarten Transceiver in der (jeweiligen) Reihe bzw. Spalte reziproke Messungen, insbesondere mit Ausnahme des ersten und letzten Transceivers der (jeweiligen) Reihe bzw. Spalte. Für den ersten und letzten Transceiver der (jeweiligen) Reihe bzw. Spalte wird bevorzugt nur mit einem Nachbar-Transceiver eine reziproke Messung durchgeführt. Wenn es sich z.B. um eine oder mehrere geschlossene Reihen von Transceivern handelt, etwa, weil die Transceiver insbesondere an einem hohlzylinderförmigen Basiskörper in einer oder mehreren (Kreis-)Reihen angeordnet sind, kann im Prinzip ein beliebiger Transceiver der geschlossenen (Kreis-)Reihe als erster Transceiver gewählt werden. Ein direkter Nachbar-Transceiver, beispielsweise der linke Nachbar dieses, ist dann insbesondere der letzte Transceiver. Für den ersten Transceiver wird dann beispielsweise nur eine reziproke Messung mit seinem rechten Nachbar-Transceiver durchgeführt und für den letzten Transceiver nur eine reziproke Messung mit seinem linken Nachbar-Transceiver. Mit anderen Worten wird die geschlossene Reihe, was die Verbindungen benachbarter Transceiver über reziproke Messungen angeht, an einer Stelle offengelassen. Da innerhalb der jeweiligen Reihe für alle Transceiver, gegebenenfalls mit Ausnahme des ersten und letzten, gilt, dass mit je zwei Nachbar-Transceivern, insbesondere dem linken und rechten Nachbarn, reziproke Messungen durchgeführt werden, wird vorliegend auch von einer "Mesh2"-Konfiguration gesprochen.

Bezüglich Spalten kann insbesondere gelten, dass der oberste oder unterste Transceiver der erste oder letzte Transceiver ist, für den dann nur mit einem Nachbar-Transceiver reziprok gemessen wird.

Um mehrere Reihen von Transceivern über reziproke Messungen miteinander zu verbinden, können zusätzlich reziproke Messungen zwischen diesen erfolgen. In weiterer bevorzugter Ausgestaltung ist daher vorgesehen, dass zwischen wenigstens zwei Transceivern, die in unterschiedlichen, insbesondere benachbarten Reihen liegen, eine reziproke Messung durchgeführt wird. Beispielsweise kann es sein, dass die Transceiver in mehreren Reihen und Spalten angeordnet sind und zwischen allen Transceivern der jeweiligen Reihe, insbesondere mit Ausnahme eines jeweils ersten und letzten Transceivers der Reihe, reziprok gemessen wird und zwischen allen Transceivern - insbesondere mit Ausnahme des ersten und letzten - mindestens einer der Spalten. Die reziproken Messungen in der wenigstens einen Spaltenrichtung verbinden dann die Reihen. Auch in dem Falle, dass die Reihen über reziproke Messungen in einer Spalte verbunden sind, wird vorliegend von "Mesh2" gesprochen.

Es kann auch zwischen den benachbarten Transceivern nicht nur einer sondern jeweils aller Spalten, optional wiederum jeweils mit Ausnahme eines ersten und letzten Transceivers der jeweiligen Spalte, reziprok gemessen werden. Dann finden für jeden Transceiver - ggf. mit den genannten Ausnahmen - insbesondere mit jedem linken, rechten, oberen und unteren Nachbar-Transceiver, also mit vier Nachbarn, reziproke Messungen statt, was vorliegend auch als "Mesh4" bezeichnet wird.

Schließlich können natürlich auch - alternativ oder zusätzlich - reziproke Messungen zwischen in diagonaler Richtung benachbarter Transceiver erfolgen. Wenn für jeden Transceiver - wiederum gegebenenfalls mit Ausnahme jeweils erster und letzter Transceiver der (jeweiligen) Reihe und/oder Spalte - gilt, dass reziproke Messungen mit dem rechten, linken, oberen und unteren sowie den vier diagonalen Nachbarn, insbesondere dem linken oberen, dem rechten oberen, dem linken unteren und dem rechten unteren Nachbar-Transceiver, also mit insgesamt acht Nachbarn, reziproke Messungen durchgeführt werden, wird vorliegend auch von "Mesh8" gesprochen.

Es sei betont, dass es sich insbesondere für die Mesh-Kalibrierung zwar als besonders geeignet erweisen hat, wenn die Trigger-Offsets von Transceiver-Paaren betrachtet bzw. herangezogen werden, die jeweils direkte Nachbarn, insbesondere in horizontaler und/oder vertikaler und/oder diagonaler Richtung, sind, dies jedoch nicht zwingend der Fall sein muss. Vielmehr können auch andere Meshes, mit anderen Worten Gitter, "aufgespannt" werden. Für den Fall von Cross-Hole-Messungen beispielsweise können auch reziproke Messungen zwischen Transceivern, die sich in verschiedenen Bohrlöchern befinden, und keine nächsten Nachbarn sind, durchgeführt bzw. herangezogen werden. Es kann entsprechend auch ein lochübegreifendes Mesh bzw. Gitter "aufgespannt" werden. Es kann auf Koppelsignale von Transceivern aus verschiedenen Bohrlöchern zurückgegriffen werden.

Unter mehreren Transceivern sind zwei oder mehr Transceiver zu verstehen. Die Anzahl der Transceiver ist dabei bevorzugt größer als zwei, besonders bevorzugt mindestens zwei-, drei- oder vierstellig. Als besonders geeignet hat es sich erwiesen, wenn eine vierstellige Anzahl von Transceivern vorgesehen ist, beispielsweise 3000 Transceiver. Insbesondere für den Fall, dass mehrere hundert oder tausend, beispielsweise 3000 Transceiver an einem Basiskörper, etwa Lysimeter, angeordnet sind bzw. werden, können EM-Wellen besonders gut aus allen Raumrichtungen in eine im Basiskörper angeordnete Probe, etwa eine darin befindliche Bodensäule, eingestrahlt werden. Wenn 3000 Transceiver vorhanden sind, können diese beispielsweise in Reihen und Spalten mit je 120 Transceivern pro Reihe und 25 Transceivern pro Spalte um den Basiskörper der bzw. der jeweiligen Radar-Einrichtung insbesondere gleichmäßig verteilt angeordnet sein.

Die Transceiver können an wenigstens einem Basiskörper gehalten sein, beispielsweise außenseitig. Es kann sich beispielsweise um einen hohlzylinderförmigen Basiskörper, beispielsweise ein Lysimeter handeln. Dies hat sich als besonders geeignet erwiesen, wenn Bodenmessungen durchgeführt werden. Ist der Basiskörper hohlzylinderförmig ausgebildet, kann er eine oder zwei offene Stirnseiten aufweisen, mit anderen Worten ein- oder beidseitig offen ausgebildet sein.

Sind die Transceiver außenseitig an einem Basiskörper gehalten, sind sie beispielsweise derart ausgebildet und positioniert, dass mittels dieser elektromagnetische Messsignale in den Basiskörper eingestrahlt werden können.

Alternativ oder zusätzlich zu einem hohlzylinderförmigen Basiskörper sind auch andere Formen möglich. Insbesondere für den Fall einer Cross-Hole-Messung über mehrere Bohrlöcher kann beispielsweise auch wenigstens ein Seil und/oder wenigstens ein Stab als Basiskörper zum Einsatz kommen, der bzw. das (jeweils) einen oder mehrere Transceiver der Einrichtung trägt.

Der Basiskörper kann aus einem Kunststoff bestehen bzw. Kunststoff umfassen. Rein bespielhaft für ein geeignetes Material für den Basiskörper sei Polyvinylchlorid (PVC) genannt.

Die zu untersuchende Probe, beispielsweise Bodenprobe, kann in dem Basiskörper angeordnet werden bzw. sein. Ein Lysimeter beispielsweise kann mit zu untersuchendem Boden befüllt werden bzw. sein. Auch ist es möglich, dass ein Basiskörper in und/oder an und/oder neben einer zu untersuchenden Probe angeordnet wird. Rein bespielhaft sei genannt, dass ein Basiskörper, an dem einer oder mehrere Transceiver gehalten sind, in ein Bohrloch im Boden eingesetzt wird, um einen das Loch umgebenden Bodenabschnitt als Probe untersuchen zu können.

Weiter bevorzugt gilt, dass die Transceiver - oder zumindest ein Teil dieser - im Wesentlichen äquidistant voneinander beabstandet angeordnet sind, insbesondere an dem Basiskörper.

In weiterer bevorzugter Ausgestaltung des erfindungsgemäßen Verfahrens fungiert jeder Transceiver einmal als Sender, während der oder alle anderen Transceiver dann als Empfänger fungieren.

Auch kann vorgesehen sein, das für alle möglichen Paare von Transceivern jeweils eine reziproke Messung mit einer Hin- und einer Rück-Messung durchgeführt wird. Natürlich können auch nur für einen Teil aller möglicher Paare reziproke Messungen durchgeführt werden.

Aus der auf die erfindungsgemäß Weise ermittelten, insbesondere berechneten Laufzeit wird in bevorzugter Ausgestaltung auf die Permittivität der Probe geschlossen. Hierfür kann auf vorbekannte Ansätze zurückgegriffen werden (siehe beispielsweise Annan 2003). Alternativ oder zusätzlich kann aus der Laufzeit auch auf die elektrische Leitfähigkeit geschlossen werden.

Wird eine Mehrzahl von Transceivern, bevorzugt eine mindestens zweistellige Anzahl, verwendet, können Tomographiemessungen erfolgen, so dass auch die räumliche Verteilung von Permittivität und/oder elektrischer Leitfähigkeit geschlossen werden kann.

Weiterhin kann vorgesehen sein, dass mittels einer Volle-Wellenform-Inversion (englisch: Full-Waveform-Inversion, kurz FWI) auf die räumliche Verteilung der Permittivität und/oder Leitfähigkeit der zu untersuchenden Probe geschlossen wird.

Insbesondere für den Fall, dass es sich bei der Probe um eine Bodenprobe handelt, kann aus der Permittivität der Wassergehalt der Bodenprobe (englisch: Soil Water Content, kurz: SWC) bestimmt werden (siehe beispielsweise Huisman et al. 2003).

In ganz besonders bevorzugter Ausgestaltung ist vorgesehen, dass die Probe über einen vorgegebenen Messzeitraum mehrfach untersucht wird. Mit anderen Worten findet über einen vorgegebenen Messzeitraum ein Monitoring der Probe mittels der erfindungsgemäßen Verfahrens statt. Zweckmäßiger Weise erfolgen dann im Rahmen jeder (Einzel-)Untersuchung die Schritte der einen oder mehreren reziproken Messung, die Ermittlung derwenigstens einen Laufzeit unter Rückgriff auf die Hin- und Rück-Ankunftszeit und des Schließens auf Eigenschaften der zu untersuchenden Probe, insbesondere einer Permittivität dieser, aus der wenigstens einen Laufzeit. Eine zur Durchführung des erfindungsgemäßen Verfahrens verwendete Radar-Einrichtung kann für ein solches Monitoring über einen vorgegebenen Zeitraum insbesondere fest installiert sein bzw. werden.

Gegenstand der Erfindung ist ferner eine Radar-, insbesondere Geo-Radar-Einrichtung, zur Durchführung des erfindungsgemäßen Verfahrens, umfassend mehrere, insbesondere 3000 Transceiver, die jeweils sowohl als Sender als auch als Empfänger für elektromagnetische Messsignale fungieren können und bevorzugt jeweils einen Sende-Schaltkreis und einen Empfangs-Schaltkreis und eine Antenne aufweisen, und ein Steuer- und Auswerte-Modul, das zur Durchführung des Verfahrens nach einem der vorhergehenden Ansprüche ausgebildet und eingerichtet ist.

Das Steuer- und Auswertemodul kann in Software oder Hardware oder einer Kombination aus Software und Hardware implementiert sein. Es ist zweckmäßiger Weise einerseits ausgebildet und eingerichtet, um die Transceiver anzusteuern, insbesondere eine Triggerung dieser zu bewirken, wobei die erfindungsgemäßen reziproken Messungen veranlasst werden können. Andererseits ist es zweckmäßiger Weise dazu ausgebildet und eingerichtet, auf die erfindungsgemäße Weise die wenigstens eine Laufzeit zu berechnen und aus dieser auf Eigenschaften der zu untersuchenden Probe zu schließen, beispielsweise eine Permittivität dieser zu ermitteln, insbesondere zu berechnen. Das Steuer- und Auswertemodul kann in Weiterbildung zur Durchführung der paarweisen Kalibrierung oder zur Durchführung der Mesh-Kalibrierung oder auch zur Durchführung sowohl der paarweisen als auch der Mesh-Kalibrierung ausgebildet sein.

In Weiterbildung der erfindungsgemäßen Radar-Einrichtung ist wenigstens ein insbesondere hohlzylinderförmiger Basiskörper, beispielsweise ein Lysimeter, vorgesehen. An dem wenigstens einen Basiskörper können die Transceiver bevorzugt außenseitig gehalten sein. Alternativ oder zusätzlich kann vorgesehen sein, dass die Transceiver in einer oder mehreren Reihen und/oder einer oder mehreren Spalten und/oder zumindest im Wesentlichen äquidistant voneinander beabstandet gehalten sind.

Die erfindungsgemäße Radar-Einrichtung kann ferner eine zentrales, der Triggerung der Transceiver dienendes Trigger-Modul umfassen. Dann gilt bevorzugt, dass das Trigger-Modul mit jedem der Transceiver über wenigstens ein Kabel direkt oder indirekt verbunden ist. Indirekt, da das Triggersignal auch durch Transceiver zu weiteren Transceivern geleitet werden kann. Dann kann weiterhin gelten, dass sich die verschiedenen Transceivern zugeordneten Kabel hinsichtlich ihrer Länge voneinander unterscheiden.

Das Trigger-Modul kann in Software oder Hardware oder einer Kombination aus Software und Hardware implementiert sein.

Auch für den Fall, dass die Radar-Einrichtung mehrere Basiskörper umfasst, an denen bevorzugt jeweils mehrere Transceiver gehalten sind, etwa um Cross-Hole-Messungen durchzuführen, kann ein zentrales Trigger-Modul vorgesehen sein, welches der Triggerung aller Transceiver dient.

Die vorliegende Erfindung stellt mit anderen Worten eine Kalibrier-Strategie des zeitlichen Nullpunktes bereit, die zum Beispiel für eine Radar-Einrichtung mit einer Mehrzahl von Transceivern, die in einer bzw. um eine Bodenprobe herum angeordnet werden, genutzt werden kann. Für den Fall, dass eine solche Einrichtung insbesondere deren Transceiver, erst nach Positionierung der Transceiver, insbesondere eines oder mehrerer dieser tragende Basiskörper, bzw. nach der Befüllung des Basiskörpers mit der Probe, insbesondere nach Befüllung des Lysimeters mit Boden, installiert wird bzw. werden kann, kann die Kalibrierung nicht mit bekanntem Material, wie etwa Luft, zwischen den Antennen zweier Transceiver durchgeführt werden.

Bevorzugt wird angenommen, dass die Transceiver komplett identisch sind, während die Trigger-Leitungen für die Kontrolle der einzelnen Transceiver unterschiedliche Längen haben können. Daher wird zweckmäßiger Weise für jede mögliche Tx/Rx-Kombination eine separate Korrektur des zeitlichen Nullpunktes durchgeführt. Der erfindungsgemäße Ansatz nutzt die Möglichkeit, jede Transceiver-Antenne als *Tx* und *Rx* verwenden zu können, und damit die Durchführung reziproker Messungen zwischen je zwei Transceiver-Antennen zu ermöglichen. Dies ermöglicht die direkte Messung der Laufzeit des Signales zwischen zwei Antennen, dies unabhängig von der Position der Antennen. Diese Vorgehensweise wird vorliegend als paarweise Kalibrierung bezeichnet.

Darüber hinaus werden reziproke Messungen insbesondere zwischen benachbarten Transceivern mit einer Superposition kombiniert, um den zeitlichen Nullpunkt für jede Tx/Rx-Kombination zu berechnen, was als Mesh-Kalibrierung bezeichnet wird. Unter Superposition ist dabei insbesondere die Addition von reziproken Messungen wie in Gleichung (11) zu verstehen.

Die beiden Kalibriermethoden nach erfindungsgemäßen Weiterbildungen eignen sich besonders für gleiche bzw. bekannte Signal-Laufzeiten in den Transceivern. Vor- und Nachteile der paarweisen und der Mesh-Kalibrierung sind in Tabelle I gegenübergestellt.

Tabelle I

| Paarweise Kalibrierung | Mesh-Kalibrierung |
|---|---|
| + einzelne reziproke Messung kann kalibriert werden | + Messungen zur Prüfung analoger Schaltkreis-Laufzeiten sind enthalten |
| - Reziprozität des Bodens zwischen Antennen ist erforderlich | + Vorkalibrierung für schnelle Messungen ist möglich |
| - längere Messzeiten für ein komplettes Tomogramm | - jeder Transceiver muss wenigstens einmal senden und empfangen |

Die Erfindung leistet einen wichtigen Beitrag zu einer insbesondere automatisierten In-Situ Korrektur des zeitlichen Nullpunktes. Die Erfindung ist dabei keineswegs auf Lysimeter-Anwendungen beschränkt, sondern kann im Prinzip für alle Radar-Einrichtungen, insbesondere -Messsysteme genutzt werden, die mehrere Transceiver umfassen, die als Sender und Empfänger fungieren können, so dass reziproke Messungen möglich sind, so dass kein spezieller Mess-Aufbau für die Kalibrierung des zeitlichen Nullpunktes benötigt wird. Man kann Radar-Einrichtungen mit mehreren Transceivern auch als Mehrkanal-Radar-Einrichtungen bezeichnen.

Auf die erfindungsgemäße Weise können prinzipiell beliebige Proben untersucht werden. Rein beispielhaft seien Bodenproben genannt, die beispielsweise jeweils durch einen Erdreichabschnitt gegeben sein können. Bei der bzw. den zu untersuchenden Proben kann es sich aber auch um beliebige andere Objekte bzw. Objektabschnitte handeln, beispielsweise um Brücken bzw. Brückenpfeiler, andere Gebäude bzw. Gebäudeabschnitte etc..

Als ein beispielhafter Anwendungsfall sei genannt, dass eine innerhalb eines Basiskörpers, insbesondere Lysimeters angeordnete Bodenprobe, mit anderen Worten Bodensäule, untersucht wird, wobei bevorzugt Tomographieaufnahmen gemacht werden.

Für den Fall von Messungen an einer Brücke können Transceiver beispielsweise an einer Ober- und Unterseite der Brücke und/oder um einen Brückenpfeiler herum angeordnet, insbesondere fest montiert werden, um das Brückenmaterial, das sich zwischen den Transceivern befindet. Zu untersuchen.

Auch in diesem Fall kann über die wenigstens eine im Rahmen des erfindungsgemäßen Verfahrens aus der/den reziproken Messungen ermittelten Laufzeit(en) auf Probeneigenschaften, insbesondere die Permittivität, geschlossen werden. Die Permittivität hängt u.a. von Lufteinschlüssen innerhalb des Proben-, etwa Brückenmaterials, ab, so dass dann aus der Laufzeit/Permittivität beispielsweise auf mögliche Risse geschlossen werden kann.

Auch für "Cross-Hole-Messungen" ist die erfindungsgemäße Vorgehensweise sehr gut geeignet. Dabei werden bevorzugt mehrere erfindungsgemäße Radar-Einrichtungen in mehreren beabstandeten Bohrlöchern angeordnet und es wird der Boden zwischen den Bohrlöchern untersucht.

Die Erfindung ist, wie erwähnt, keineswegs auf Untersuchungen des Bodens beschränkt. Vielmehr kann eine oder können mehrere erfindungsgemäße Radar-Einrichtungen auch an beliebigen anderen Objekten zum Einsatz kommen, insbesondere, um ein Monitoring durchzuführen. Rein beispielhaft sei die Überwachung von Brücken, etwa auf Risse und dergleichen, genannt.

Das erfindungsgemäße Verfahren kann in vorteilhafter Weiterbildung softwaregestützt ausgeführt werden. Es kann insbesondere wenigstens eine Software zum Einsatz kommen, mittels derer die Schritte des erfindungsgemäßen Verfahrens durchgeführt werden.

Ein weiterer Gegenstand der Erfindung ist entsprechend ein Computerprogramm, umfassend Befehle, die bei der Ausführung des Programms durch einen Computer diesen veranlassen, die Schritte des erfindungsgemäßen Verfahrens auszuführen.

Gegenstand der Erfindung ist auch ein Computerprogrammprodukt, umfassend Befehle, die bei der Ausführung des Programms durch einen Computer diesen veranlassen, die Schritte des erfindungsgemäßen Verfahrens auszuführen.

Due reziproken Messungen des erfindungsgemäßen Verfahrens können softwaregesteuert automatisiert durchgeführt werden, und die Auswertungen zum Erhalt der wenigstens einen Laufzeit und davon abgeleiteten Probeneigenschaft(en) können softwarebasiert automatisiert erfolgen.

Die erfindungsgemäße Radar-Einrichtung kann einen Computer umfassen, der beispielsweise ein Auswerte- bzw. Steuer- und Auswertemodul der Vorrichtung bildet bzw. Bestandteil eines solchen ist. Das erfindungsgemäße Computerprogramm kann dann auf den Computer bzw. das Auswerte- bzw. Steuer- und Auswertemodul der Vorrichtung geladen sein oder werden, um das Verfahren mittels der Vorrichtung durchzuführen. Sowohl Ansteuerung der Transceiver für die reziproken Messungen als auch Auswertungen zum Erhalt der wenigstens einen Laufzeit und davon abgeleiteter Größen, beispielsweise eine Permittivität der Probe, können durch das erfindungsgemäße Computerprogramm erfolgen.

Hinsichtlich der Ausgestaltungen der Erfindung wird auch auf die Unteransprüche sowie auf die nachfolgende Beschreibung von Ausführungsbeispielen unter Bezugnahme auf die beiliegende Zeichnung verwiesen.

In der Zeichnung zeigt:

Figur 1 eine rein schematische Darstellung eines Ausführungsbeispiels einer erfindungsgemäßen Radar-Einrichtung mit einem zylinderförmigen Basiskörper, der im Horizontalschnitt gezeigt ist;

Figur 2 die Radar-Einrichtung aus Figur 1 in rein schematischer Darstellung, wobei ein Vertikalschnitt durch den Basiskörper gezeigt ist;

Figur 3 einen Graphen, der wichtige Zeiten einer Messung veranschaulicht;

Figur 4 in rein schematischer Darstellung drei beispielhafte Topologien für reziproke Messungen für eine Mehrzahl von Transceivern, die in Reihen und Spalten angeordnet sind;

Figur 5 einen Graphen mit Koppelsignalen für variierende Tx/Rx-Konfigurationen;

Figur 6 einen Graphen, der Weitwinkel-Reflexions- und -BrechungsMessungen in Luft zeigt;

Figur 7 normalisierte Quadratmittelwerte (nRMS) für die drei Topologien aus Figur 4;

Figur 8 gemittelte nRMS-Werte $\overline{nRMS_i}$ für jede Spalte i aus Figur 7 für die drei Topologien gemäß Figur 4;

Figur 9 Koppelsignalen, wenn sich entweder Luft oder Wasser hinter der PVC-Platte des Testaufbaus befand für Antenne 6 als *Tx* und (a) Antenne 7 als *Rx* (rechter Nachbar) bzw. (b) Antenne 10 als *Rx* (unterer Nachbar) bzw. (c) Antenne 11 als *Rx* (rechter unterer diagonaler Nachbar);

Figur 10 Zeit-Verzögerungen in Pikosekunden für Luft- und Wassermessungen und für variierende Rx-Antennen; und

Figur 11 ein rein schematisches Blockdiagramm mit den Schritten eines Ausführungsbeispiels des erfindungsgemäßen Verfahrens.

In den Figuren sind gleiche bzw. analoge Komponenten/Elemente mit gleichen Bezugszeichen versehen.

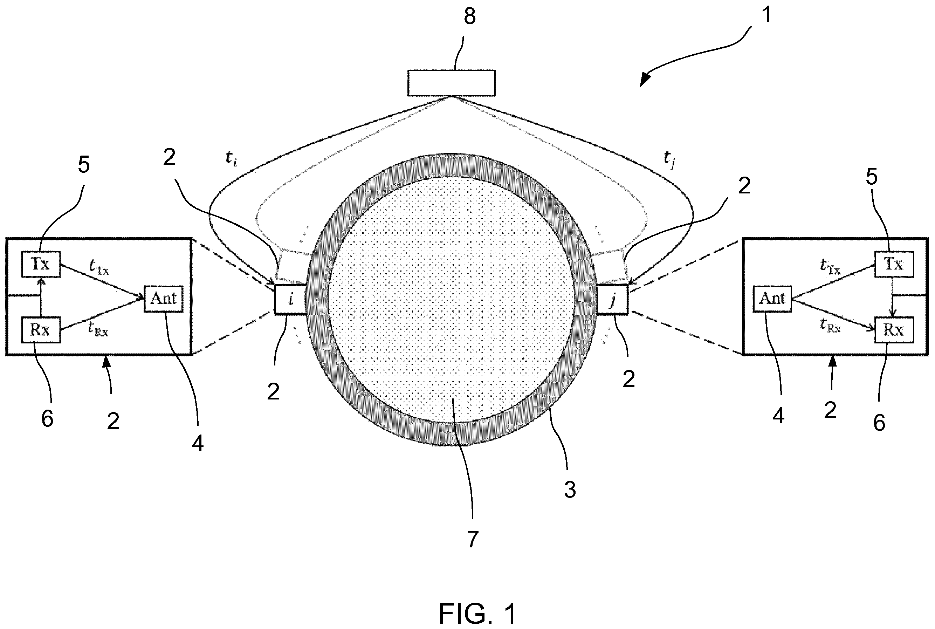

Die Figur 1 zeigt ein Ausführungsbeispiel einer erfindungsgemäßen Radar-Einrichtung 1. Diese umfasst eine Mehrzahl von Transceivern 2, die jeweils sowohl als Sender als auch als Empfänger für elektromagnetische (EM) Messsignale fungieren können, und die an einem hohlzylinderförmigen Basiskörper 3 der Einrichtung 1 außenseitig gehalten sind. Es sind insgesamt 3000 Transceiver 2 vorgesehen, wobei diese Anzahl rein beispielhaft zu verstehen ist.

Jeder Transceiver 2 umfasst oder besteht aus einer Antenne 4, einem Tx-Schaltkreis 5, der einen Signalgenerator umfasst und einem Rx-Schaltkreis 6. Der Signalgenerator erzeugt das Messsignal, des über den TX-Pfad an die Antenne 4 geleitet wird. Die Figur 1 enthält links neben dem linken schwarz hervorgehobenen Transceiver 2 sowie rechts neben dem rechten schwarz hervorgehobenen Transceiver 2 auch vergrößerte Darstellung dieser, welche deren innere Struktur bzw. Aufbau rein schematisch andeuten. Hier sind auch die Antenne 4, der Tx-Schaltkreis 5 und der Rx- Schaltkreis 6 des jeweiligen Transceivers schematisch 2 dargestellt. Weiterhin eingezeichnet sind hier die analogen Transceiver Schaltkreis Laufzeiten $t_{Tx}$ und $t_{Rx}$, die für alle Transceiver 2 gleich sind bzw. sich in nur vernachlässigbarer Weise, um weniger als 5 ps voneinander unterscheiden. $t_{Tx}$ und $t_{Rx}$ beschreiben die Zeiten, die ein Puls braucht, um in dem *Tx* bzw. *Rx* Schaltkreis zu propagieren, diesen mit anderen Worten zu durchlaufen.

Die Antennen 4 der Transceiver 2 sind jeweils nahe der Wandung des Basiskörper 3 angeordnet und zeigen zu dieser.

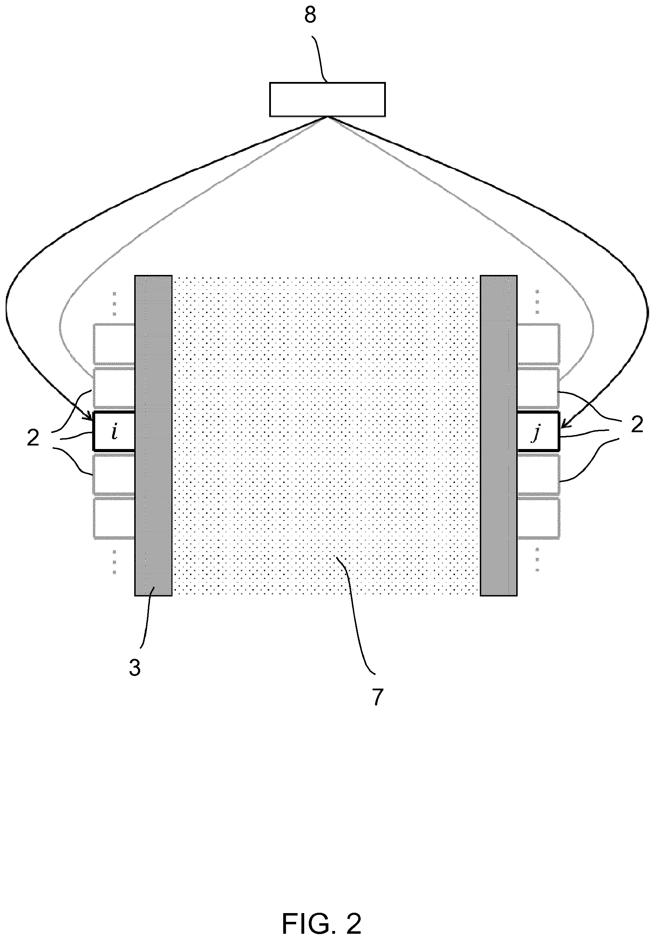

Alle Transceiver 2 sind baugleich ausgebildet. Die Transceiver sind hier außenseitig an dem Lysimeter angeordnet und gleichmäßig - in der Art eines regelmäßigen Gitters - in mehreren Reihen und Spalten um dieses verteilt. Konkret sind je 120 Transceiver pro Reihe und 25 Transceiver pro Spalte um den Basiskörper 3 angeordnet. In Figur 1, welche einen Horizontalschnitt zeigt, ist eine der mehreren Reihen von Transceivern angedeutet, wobei nur 4 der insgesamt 120 Transceiver 2 dargestellt und die verbleibenden mit "..." nur angedeutet sind. In der Figur 2, welche einen Vertikalschnitt zeigt sind zwei der Spalten von Transceivern erkennbar, wobei auch hier gilt, dass nur einige, konkret 5 der insgesamt 25 übereinanderliegenden Transceiver 2 der jeweiligen Spalte dargestellt und die weiteren durch "..." nur angedeutet sind.

Die Radar-Einrichtung mit einer großen Anzahl, hier 3000 um den Basiskörper 3 verteilten Transceiver 2 ermöglicht es, EM-Wellen aus allen Raumrichtungen in eine in dem Basiskörper 3 angeordnete Probe, etwa eine darin befindliche Bodensäule, zu strahlen, ähnlich wie z.B. bei Stoffregen et al. 2002 und Schmalholz et al. 2004

Bei dem Ausführungsbeispiel ist der Basiskörper 3 durch ein Lysimeter (mit anderen Worten eine zylindrische Schale, verwendet z.B. von Hannes et al. 2015 und Pütz et al. 2016) gegeben, welches mit einer Bodenprobe 7 als zu untersuchender Probe befüllt werden kann bzw. befüllt ist. Das Lysimeter 3 besteht hier aus Polyvinyl Chlorid (PVC), hat eine Höhe von 1,5 m, eine innere Querschnittsfläche von 1 $m^2$ und eine Wandstärke von 0,03 m. Ein Trigger-Modul 8, welches auch als Master-Modul bezeichnet werden kann, wird zur Triggerung der Transceiver 2 verwendet, wobei $t_i$ die Zeit definiert, die der Trigger braucht, um beim entsprechenden Transceiver *i* anzukommen. In der Figur 1 sind die Trigger-Zeiten $t_i$ und $t_j$ für die in der Figur mit i und j bezeichneten beiden Transceiver 2 ebenfalls dargestellt und zwar schematisch über das Trigger-Modul 8 mit dem jeweiligen Transceiver 2 verbindende Pfeile. Das Trigger-Modul 8 steuert die Transceiver 2 und ist bei dem dargestellten Ausführungsbeispiel Bestandteil eines nicht weiter dargestellten zentralen Steuerund Auswertemoduls der Einrichtung 1. Vorliegend handelt es sich bei dem Trigger-Modul 8 um das Model HMC7044 des Unternehmens Analog Devices, wobei dies rein beispielhaft zu verstehen ist.

Für 3000 Transceiver 2 markiert t = $(t_1, ..., t_{3000})^T$ die Triggerzeiten aller Transceiver.

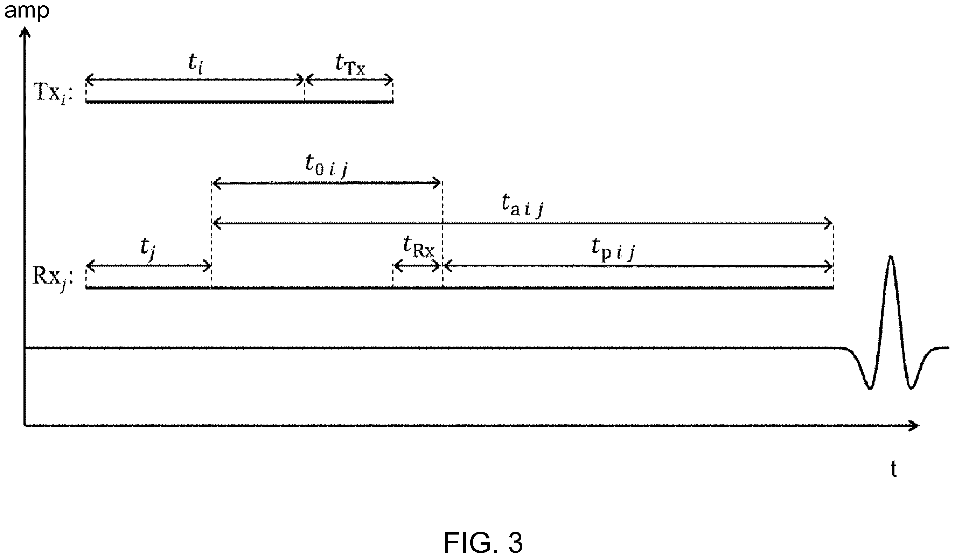

Die Figur 3 zeigt - rein schematisch - wichtige Zeitsegmente für eine Messung zwischen zwei Transceivern *i* und *j* In dem Graphen ist (einheitenlos) die Amplitude amp über der Zeit t aufgetragen. $t_i$ und $t_j$ sind die Trigger-Zeiten der Transceiver *i* und *j* $t_{Tx}$ und $t_{Rx}$ sind die analogen Schaltkreis Laufzeiten. Diese beschreiben die Zeiten, die ein Puls braucht, um in dem Tx- bzw. Rx-Schaltkreis 5, 6 zu propagieren, diesen mit anderen Worten zu durchlaufen. $t_{a\,ij}$ beschreibt die Ankunftszeit des Signals am Empfänger, konkret der Empfänger-Antenne 4, wenn Transceiver *i* sendet und Transceiver *j* empfängt, $t_{a\,j}$ *die* Ankunftszeit für den umgekehrten Fall. $t_{p\,ij}$ ist die Laufzeit des Signals von der sendenden Antenne 4 von Transceiver *i* zu der empfangenden Antenne 4 von Transceiver *j*, $t_{pj}$ wiederum für den umgekehrten Fall.

Die Zeitachse beginnt mit der Aussendung des Triggers am Trigger-Modul 8. Der Signalgenerator des sendenden Transceivers i beginnt zum Zeitpunkt $t_i$, zum Zeitpunkt $t_i$ startet mit anderen Worten der Tx-Schaltkreis 5, während die Datenerfassung des Empfangs-Transceivers *j* zum Zeitpunkt $t_j$ beginnt. Das Signal wird von der Antenne *i* zum Zeitpunkt $t_i + t_{Tx}$ in das Lysimeter 3 emittiert. In Figur 3 sind die Zeiten umsortiert, um den Zusammenhang zwischen dem zeitlichen Nullpunkt und den Triggerzeiten der entsprechenden Transceiver zu verdeutlichen. Die Lücke zwischen $t_j$ und $t_{Rx}$ veranschaulicht, dass die Datenerfassung von Transceiver *j* startet, bevor das Signal von Transceiver *j* emittiert wird. Es ist zu beachten, dass eine Änderung der Reihenfolge von $t_{Rx}$ und $t_{p\,ij}$ *die* Ankunftszeit nicht ändert. $t_{0\,ij}$ ist schließlich der zeitliche Nullpunkt, wenn *i* sendet und *j* empfängt.

Unter Verwendung der Definition von $t_0 = t_a - t_p$, wobei $t_p$ die Laufzeit des Signals zwischen den Antennen ist, erhält man die obige Gleichung (3). Gleichung (4) definiert das Trigger-Offset der Transceiver *i* und *j* als $t_{ij} := t_i - t_j$. Somit setzt die Bestimmung des zeitlichen Nullpunktes für ein beliebiges Transceiver-Paar die Kenntnis der Differenz zwischen den Triggerzeiten der entsprechenden Transceiver 2 voraus. Die Triggerzeiten unterscheiden sich vor allem durch unterschiedliche Kabellängen zu den Transceivern 2. Wenn $t_{Tx}$ und $t_{RX}$ für alle 3000 Transceiver gleich - bzw. in nur vernachlässigbarer Weise, um weniger als 5 ps verschieden und stabil sind, müssen diese Zeiten nur einmal kalibriert werden. Dies ist sinnvoll, weil das Systemdesign darauf abzielt, gleich gebaute Transceiver 2 mit im Vergleich zu den Trigger-Zeiten vernachlässigbaren Unterschieden in $t_{Tx}$ und $t_{Rx}$ zu haben.

Unbekannte Bodeneigenschaften und damit Signalgeschwindigkeiten zwischen beliebigen Antennenpaaren verhindern zunächst die Verwendung von direkten Signalen wie in obiger Gleichung (1) für eine Korrektur des zeitlichen Nullpunktes. Es ist zu beachten, dass in den meisten Fällen das Lysimeter 3 mit Bodenproben aufgefüllt wird, so dass die Transceiver 2 erst nach dem Auffüllen installiert werden können.

Unter Durchführung des im Folgenden beschriebenen Ausführungsbeispiel des erfindungsgemäßen Verfahrens zur zerstörungsfreien Untersuchung einer Probe, vorliegend Bodenprobe, kann dieses Problem überwunden werden.

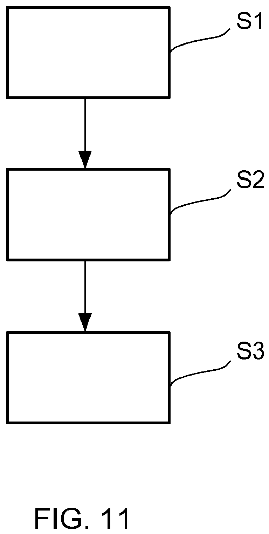

Gemäß diesem werden eine oder mehrere reziproke Messungen zwischen Transceiver-Paaren durchgeführt (Schritt S1, vgl. Figur 11). Eine reziproke Messung schließt dabei jeweils eine Hin- und eine Rück-Messung ein, wobei für die Hin-Messung der eine Transceiver 2 des oder des jeweiligen Paares als Sender und der andere Transceiver 2 als Empfänger fungiert, und für die Rück-Messung der andere Transceiver 2 des oder des jeweiligen Paares als Sender und der eine Transceiver 2 als Empfänger fungiert.

Es wird unter Rückgriff auf die oder die jeweilige Ankunftszeit des Messsignals an dem einen Transceiver 2, insbesondere einer Antenne 4 dieses, während der oder der jeweiligen Hin-Messung, Hin-Ankunftszeit $t_{a\,i\,j}$, und der Ankunftszeit des Messsignals an dem anderen Transceiver 2, insbesondere einer Antenne 4 dieses, während der oder der jeweiligen Rück-Messung, Rück-Ankunftszeit $t_{a\,j\,i}$, wenigstens eine Laufzeit $t_p$ des Messsignales zwischen den beiden Transceivern 2 des bzw. des jeweiligen Paares ermittelt (Schritt S2, vgl. Figur 11).

Aus der wenigstens einen Laufzeit $t_p$ wird auf Eigenschaften der zu untersuchenden Probe, hier des in dem Lysimeter 3 angeordneten Bodens 7, insbesondere eine Permittivität dieser, geschlossen (Schritt S3, vgl. Figur 11).

Für eine komplette Tomographie-Messung der Bodenprobe 7 wird zweckmäßiger Weise jeder Transceiver 2 einmal zum Senden verwendet, während die anderen 2999 Transceiver als Empfänger dienen.

Bei dem dargestellten Beispiel wird ferner für jede reziproke Messung die Laufzeit $t_p$ zwischen den Antennen 4 des jeweiligen Transceiver-Paares ermittelt.

Das zentrale Steuer- und Auswertemodul der Einrichtung 1 ist zur Durchführung des Verfahrens ausgebildet. Es umfasst vorliegend einen Computer, auf den ein Ausführungsbeispiel eines erfindungsgemäßen Computerprogrammes geladen ist, mittels dem die Schritte des Verfahrens ausgeführt werden können.

Besonders bevorzugt wird im Rahmen des erfindungsgemäßen Verfahrens ein paarweiser und/oder ein Mesh-Kalibrieransatz genutzt.

Gemäß dem Ansatz der paarweisen Kalibrierung gilt, dass die Laufzeit $t_p$ zwischen einem Transceiver $i$ und einem Transceiver jeines Transceiver-Paares mittels der obigen Formel (7) bestimmt wird, wobei $t_{a\,i\,j}$ die Hin-Ankunftszeit, $t_{a\,j\,i}$ die Rück-Ankunftszeit, $t_{Tx}$ die analoge Sendepfad-Laufzeit und $t_{Rx}$ die analoge Empfangspfad-Laufzeit des jeweiligen Transceivers 2 ist (vgl. auch die Figuren 1 und 3). Zweckmäßiger Weise wird dabei davon ausgegangen, dass die Summe von $t_{Tx}$ und $t_{Rx}$ bekannt ist.

Dies verdeutlicht, dass die unbekannte Laufzeit $t_p$ des Signals zwischen $i$ und $j$ durch das unbekannte Medium, die normalerweise nur über einen kalibrierten zeitlichen Nullpunkt bestimmt werden kann, direkt durch eine reziproke Messung ermittelt werden kann. Dies insbesondere, wenn Signallaufzeit innerhalb der Analogschaltung $t_{Tx} + t_{Rx}$, bekannt ist. Der Vorteil dieser Methode ist, dass jedes einzelne Transceiver-Paar unabhängig von seiner Position kalibriert werden kann und dass $t_{Tx} + t_{Rx}$ nur einmal kalibriert werden muss, dies unter der Annahme, dass es sich um einen zeitlich konstanten Wert für alle Transceiver-Paare handelt.

Bei Bodenuntersuchungen beispielsweise kann es sein, dass die Reziprozität für Messungen durch die Probe unter bestimmten Bodenbedingungen verletzt ist, wie z. B. beim Vorhandensein von Wellenleitern mit niedriger Geschwindigkeit, z. B. einem Neigungswinkel oder diskontinuierlichen Schichten (Klotzsche, van der Kruk, et al. 2014). Daher werden die reziproken Messungen in bevorzugter Ausgestaltung verglichen und nur dann zur Kalibrierung verwendet, wenn die Signalformen gleich sind. Um schnelle Bodenveränderungen sichtbar zu machen, sind schnellstmögliche Tomographiemessungen zweckmäßig. In diesen Fällen kann die paarweise Kalibrierung den Nachteil haben, dass jede Signalspur zweimal in die Messdaten aufgenommen werden muss, um reziproke Messungen zu erhalten. Wenn statische Bodenbedingungen während der gesamten Messung angenommen werden, ist die reziproke Spur redundant und erhöht die Messzeit für ein vollständiges Tomogramm. Andererseits können dynamische Bewässerungsprozesse die Zeitinvarianz des Bodens stören $(t_{p\,i\,j} \neq t_{p\,j\,i})$ und könnten bei Verwendung von (7) womöglich zu nicht vollständig korrekten Ergebnissen führen.

Die paarweise Kalibrierung ist besonders gut geeignet für passive lineare zeitinvariante Systeme, die ein reziprokes Verhalten zeigen. In diesen Szenarien ist die paarweise Kalibrierung die besonders bevorzugte Methode. Wenn jedoch die Reziprozität für ein oder mehrere Transceiver-Paare nicht gegeben ist, oder wenn schnelle Messungen erforderlich sind, kann in vorteilhafter Weiterbildung die Kalibrierung erweitert bzw. durch den im Folgenden beschriebenen Mesh-Kalibrierungsansatz ergänzt oder auch ersetzt werden.

Die Mesh-Kalibrierung zielt auf die Bestimmung von $t_p$ über einmalig ermittelte zeitliche Nullpunkte ab und basiert auf einer Überlagerung von reziproken Messungen vorliegend zwischen benachbarten Transceivern 2. Dadurch kann der Einfluss der Probe, etwa des nahen Bodens 7, auf das Signal in der Radar-Einrichtung 1 minimiert werden.

Gemäß dem Mesh-Kalibrieransatz wird die Laufzeit $t_{p\,i\,j}$ zwischen einem Transceiver $i$ und einem Transceiver $j$ eines Transceiver-Paares bestimmt mittels der obigen Formel (15), wobei $t_{a\,i\,j}$ die Hin-Ankunftszeit, $t_{Tx}$ die analoge Laufzeit des Sende-Schaltkreises und $t_{Rx}$ die analoge Laufzeit Empfangs-Schaltkreises des jeweiligen Transceivers 2 ist. Es wird insbesondere davon ausgegangen, dass die Summe von $t_{Tx}$ und $t_{Rx}$ bekannt sind. Vorliegend ist die Summe ferner für alle Transceiver 2 gleich, wobei dies optional ist. $t_{i\,j}$ ist das Trigger-Offset der Transceiver $i$ und $j$ eines Transceiver-Paares, das definiert ist durch Gleichung (4), mit der Trigger-Zeit $t_i$ des Transceivers $i$ und der Trigger-Zeit $t_j$ des

Transceivers j.

Besonders bevorzugt gilt, dass die Trigger-Offsets berechnet werden mittels der vorstehenden Formel (8).

Wie oben dargelegt, hat sich im Rahmen des Mesh-Kalibrierungsansatzes die Verwendung von Matrizen als besonders geeignet erwiesen. Bevorzugt ist daher vorgesehen, dass Trigger-Offsets $t_{ij}$ von Transceiver-Paaren als Input für die Formel (15) unter Verwendung zweier Matrizen ermittelt werden. Dann gilt bevorzugt, dass eine Matrix A definiert wird, so dass $A\,t = t_{\text{measured}}$ gilt, wobei t die Trigger-Zeiten mehrerer, insbesondere aller Transceiver umfasst, und $t_{\text{measured}}$ ein Vektor ist, der die Trigger-Offset von in vertikaler und/oder horizontaler und/oder diagonaler Richtung direkt benachbarter Transceiver-Paare umfasst, die über $t_{i\,j} = \dfrac{t_{a\,i\,j} - t_{a\,j\,i}}{2}$ ermittelt werden, und $B\,t = t_{\text{searched}}$ gilt, wobei die weitere Matrix B gesuchte Transceiver-Kombinationen umfasst, und der Vektor $t_{\text{searched}}$ gesuchte Trigger-Offsets enthält, und $t_{\text{searched}} = bB\,A^+ t_{\text{measured}}$ gilt, wobei $A^+$ die Pseudoinverse der Matrix A ist, die bevorzugt durch QR-Faktorisierung oder Singulärwert-Zerlegung berechnet wird.

Es ist zu beachten, dass die Verwendung von Koppelsignalen für die Kalibrierung keine zusätzlichen Messungen während einer Tomographie erfordert, da jeder Transceiver 2 einmal als *Tx* für eine komplette Tomographie verwendet wird. Der einzige zusätzliche Aufwand besteht darin, benachbarte Transceiver 2 eines Tx-Transceivers als Rx-Transceiver zu verwenden.

Die Mesh-Kalibrierung kann beispielsweise vor Bewässerungsprozessen genutzt werden, um eine Korrektur des zeitlichen Nullpunktes basierend auf Formel (3) für beliebige zwei Transceiver 2 zu erzielen. Wenn der Boden dann schnelle Änderungen erfährt, ist es ausreichend, wenn die Hälfte aller Transceiver 2 als *Tx* für die Bodenmessung verwendet werden und demgemäß kann die Aufnahmezeit reduziert werden. Im Gegensatz zu dem paarweisen Kalibrier-Verfahren müssen bei der die Mesh-Kalibrierung nur einmal Kalibrierdaten bestimmt werden.

Timing-Fehler Überlegungen können sowohl für die paarweise als auch für die Mesh-Kalibrierung vorteilhaft sein. Es kann prinzipiell zwischen zwei Arten von Timing-Fehlerquellen unterschieden werden, nämlich systematischen und zufälligen Unterschieden zwischen $t_{Tx}$ und $t_{Rx}$ bei verschiedenen Transceivern 2. Während die Auswirkung von zufälligen Fehlern, nämlich Jitter, durch ein Stacking der Signale reduziert werden kann, werden systematische Differenzen durch das Stacking nicht beeinträchtigt. Unter Stacking ist dabei zu verstehen, dass Messungen wiederholt durchgeführt und anschließend der Mittelwert aus den mehreren Messungen berechnet und zugrunde gelegt wird. Um Trigger-Offsets von Transceivern 2 basierend auf reziproken Messungen, wie in (8), messen zu können, sollten zweckmäßiger Weise gleiche bzw. in nur vernachlässigbarem Maße, insbesondere um weniger als 5 ps verschiedene, analoge Schaltkreis-Laufzeiten $t_{Tx}$ und $t_{Rx}$ vorliegen. Jede Ungleichheit der Laufzeiten der Analogschaltungen kann zu systematischen Fehlern sowohl bei der paarweisen als auch bei der Mesh-Kalibrierung führen. Im Folgenden werden die Auswirkungen von zufälligen Timing-Fehlern auf die Trigger-Offsets mit Hilfe von Simulationen analysiert.

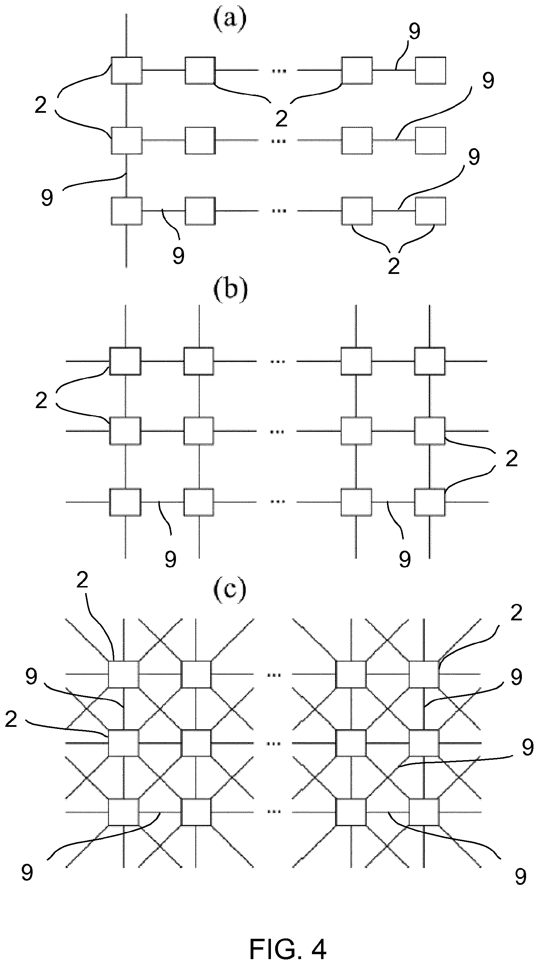

Drei bespielhafte Messtopologien für die Durchführung reziproker Messungen von Nachbar-Transceivern 2 sind rein schematisch in Figur 4 dargestellt. Diese werden im Folgenden als Mesh2 (vgl. Figur 4a), Mesh4 (vgl. Figur 4b) und Mesh8 (vgl. Figur 4c) bezeichnet. Es sei betont, dass diese drei Topologien rein beispielhaft zu verstehen und alternativ oder zusätzlich andere Topologien möglich sind. In Figur 4 repräsentiert jedes Quadrat einen Transceiver 2 beispielsweise der Radar-Einrichtung 1 gemäß den Figuren 1 und 2. Jede Linie 9 zwischen zwei Transceivern 2 repräsentiert ein gemessenes Trigger-Offset zwischen den korrespondierenden, benachbarten Transceivern 2. Von der Vielzahl der dargestellten Transceiver 2 und Verbindungslinien 9 sind in Figur 4 nur einige beispielhaft mit entsprechendem Bezugzeichen versehen.

Für Mesh2 (vgl. Figur 4a) führt jeder Transceiver 2, mit Ausnahme des letzten Transceivers 2 in jeder Reihe, eine reziproke Messung mit seinem linken und rechten Nachbar-Transceiver 2 aus, wodurch Reihen verbundener Transceiver entstehen. Um Reihen miteinander zu verbinden, wird ferner für zwei vertikal benachbarte Transceiver 2 eine reziproke Messung durchgeführt (vgl. Figur 4a ganz links).

Für Mesh4 (vgl. Figur 4b) führt jeder Transceiver eine reziproke Messung mit seinem linken, rechten, oberen und unteren benachbarten Transceiver 2 durch.

Für Mesh8 (vgl. Figur 4c) werden die vier diagonalen Nachbarn zusätzlich zu Mesh4 genutzt.

Während die Verwendung von Mesh2 ausreichend ist, um Gleichung (14) zu lösen, werden Mesh4 und Mesh8 eingeführt, um den Einfluss des Jitters durch Nutzung eines größeren Systems von linearen Gleichungen zu reduzieren. Die 3000 Transceiver 2 gemäß dem Ausführungsbeispiel sind, wie angemerkt, in 120 Spalten um das Lysimeter und 25 Zeilen übereinander aufgeteilt.

Gleichungen (16) und (17) werden verwendet, um zufällige Fehlereffekte zu implementieren:

$$t_i + t_{\text{Tx}} + \xi_{i\,\text{Tx}} + t_{p\,i\,j} + t_{\text{Rx}} + \xi_{j\,\text{Rx}} - t_j = t_{a\,i\,j} \qquad (16)$$

und

$$t_j + t_{\mathrm{Tx}} + \xi_{j\ \mathrm{Tx}} + t_{\mathrm{p}\ j\ i} + t_{\mathrm{Rx}} + \xi_{i\ \mathrm{Rx}} - t_i = t_{\mathrm{a}\ j\ i,} \qquad (17)$$

wobei $\xi_i$ und $\xi_j \sim N(0, \eta^2)$ Fehler darstellen, die von den Transceivern $i$ und $j$ eingeführt werden, entweder als Txoder als *Rx*. Das Subtrahieren der fehlerhaften Messungen und Umsortieren ergibt

$$t_{i\ j} + \underbrace{\frac{\xi_{i\ \mathrm{Tx}} - \xi_{j\ \mathrm{Tx}} + \xi_{j\ \mathrm{Rx}} - \xi_{i\ \mathrm{Rx}}}{2}}_{\xi_{i\ i}} = \underbrace{\frac{t_{\mathrm{a}\ i\ j} - t_{\mathrm{a}\ j\ i}}{2}}_{\tilde{t}_{i\ i}}, \qquad (18)$$

wobei $\xi_{ij}$ eine neue Zufallsvariable mit $\xi_{ij} \sim N(0, \eta^2)$ ist, wenn die Rechenregeln für normalverteilte Zufallsvariablen beachtet werden. Daher werden die wahren Trigger-Offsets $t_{measured}$ in (14) ersetzt durch

$$\tilde{t}_{\mathrm{measured}} = t_{\mathrm{measured}} + \xi. \qquad (19)$$

Elemente im Fehlervektor $\xi$ sind nicht unbedingt unabhängig, denn wenn ein Transceiver 2 sendet und mehrere Transceiver 2 empfangen, wirkt sich ein Fehler in $t_{Tx}$ auf alle empfangenden Transceiver 2, mit anderen Worten Empfänger, aus.

Die gleiche Berechnung kann für die Summierung fehlerhafter reziproker Messungen durchgeführt werden, wie es für die paarweise Kalibrierung zweckmäßig ist, was wiederum zu einem Fehlerterm $\xi_{ij} \sim N(0, \eta^2)$ führt:

$$t_{\mathrm{p}} = \frac{t_{\mathrm{a}\ i\ j} + t_{\mathrm{a}\ j\ i}}{2} - (t_{\mathrm{Tx}} + t_{\mathrm{Rx}}) - \frac{\xi_{i\ \mathrm{Tx}} + \xi_{j\ \mathrm{Tx}} + \xi_{j\ \mathrm{Rx}} + \xi_{i\ \mathrm{Rx}}}{2}. \qquad (20)$$

Es folgt, dass die paarweise Kalibrierung die Standardabweichung einer einzelnen Messung ($\eta$) zeigt, die durch wiederholte Messungen (Stacking) reduziert werden kann. Ein Stacking-Faktor s würde schließlich die zufällige Fehler-Standardabweichung um einen Faktor $\sqrt{s}$ reduzieren, was auch für die Mesh-Kalibrierung gilt.

Für die Simulation wurde die Matrix B in (14) so definiert, dass alle möglichen 3000 * 2999 Trigger-Offsets berechnet wurden. Abschließend kann man die Ergebnisse durch die Berechnung des normalisierten Quadratmittelwertes (englisch: normalized Root Mean Square, kurz nRMS) der berechneten fehlerhaften Trigger-Offsets $\tilde{t}_{earched}$ zwischen zwei beliebigen Transceivern $i$ und j unter Verwendung der wahren Trigger-Offsets $t_{searched}$ als

$$nRMS_{i\ j} = \frac{1}{\eta} \sqrt{\frac{\sum(t_{\mathrm{searched}} - \tilde{t}_{\mathrm{searched}})^2}{N}} \qquad (21)$$

bewerten, wobei $\eta$ die vorgegebene Standardabweichung der Zufallsfehler ist und *N d*ie Anzahl von Simulationen, die mit demselben Wert für $\eta$ durchgeführt wurden. Dies erlaubt es zu untersuchen, wie die einzelne Standardabweichung $\eta$ sich auf die gesuchten Trigger-Offsets überträgt. Neben dem Stacking der Daten durch Wiederholung der Messungen, um $\tilde{t}_{measured}$ zu erhalten, werden zufällige Fehler darüber hinaus reduziert, indem man reziproke Messungen zwischen so vielen benachbarten Transceivern 2 wie möglich durchführt.

Im Folgenden wird auf die Erkennung von systematischen Zeitfehlern eingegangen.

Es wird ein In-Situ Messansatz etabliert, um die Voraussetzung gleicher analoger Laufzeiten $t_{Tx}$ und $t_{Rx}$ zu überprüfen. Um systematische Zeitfehler zu erkennen, wird Gleichung (7) verwendet. Dies ist unter der Bedingung möglich, dass $t_p$ für alle Koppelsignale gleich ist. Als Koppelsignale werden, wie angemerkt, Signale bezeichnet, die sich zwischen benachbarten Transceivern, konkret deren Antennen, ausbreiten. Wenn gilt, dass $t_p$ für alle Kopplungssignale gleich ist, dann ist $t_{a\ ij} + t_{a\ ji}$ in Gleichung (7), wobei i und j nun benachbarte Transceiver 2 sind, für alle reziproken Messungen gleich, solange $t_{Tx} + t_{Rx}$ für alle Transceiver 2 gleich ist. Das bedeutet, dass $t_{a\ ij} + t_{a\ ji}$ ein Maß ist, um zu kontrollieren, ob $t_{Tx} + t_{Rx}$ bei allen Transceivern 2 gleich ist. Wenn z. B. Kopplungsmessungen zwischen den Transceivern 1-2 und 2-3 durchgeführt werden, gilt

$$\frac{t_{a\,1\,2}+t_{a\,2\,1}}{2} - (t_{\mathrm{Tx}} + t_{\mathrm{Rx}}) = \frac{t_{a\,2\,3}+t_{a\,3\,2}}{2} - (t_{\mathrm{Tx}} + t_{\mathrm{Rx}}) \qquad (22)$$

solange $t_p$ für beide Messungen gleich ist. Eine Abweichung von $t_{a\,1\,2} + t_{a\,2\,1}$ und $t_{a\,2\,3} + t_{a\,3\,2}$ zeigt eine Verletzung der Bedingung an, dass $t_{Tx} + t_{RX}$ für die Transceiver 2 gleich ist. Diese Berechnung ist nur gültig, wenn die Laufzeit des Koppelsignals $t_p$ nicht von der Lysimeterfüllung abhängt. Es ist zu beachten, dass zwischen drei Laufzeiten für Koppelsignale unterschieden werden muss, nämlich für horizontale, vertikale und diagonale Kopplungssignale (vgl. auch Figur 4).

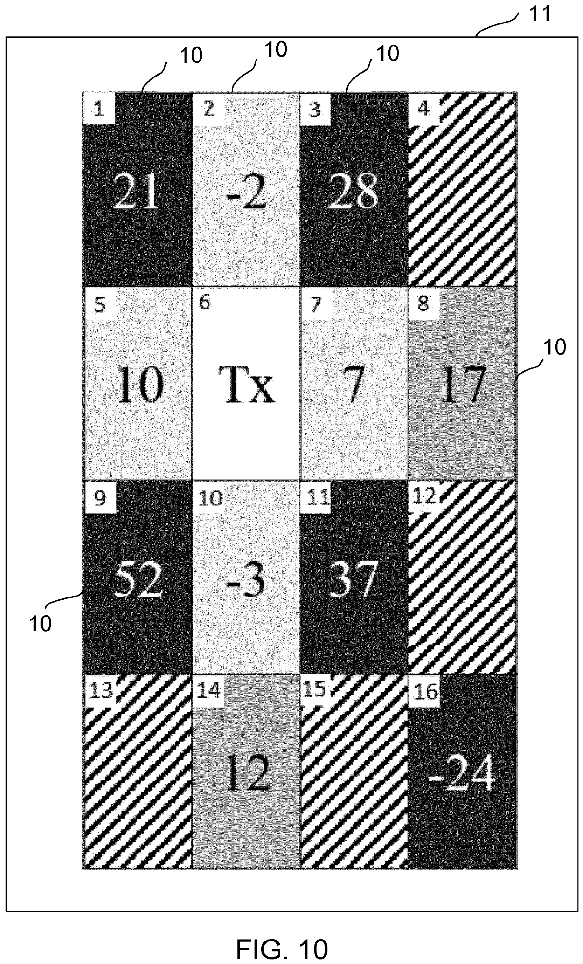

Um den Einfluss der Lysimeterfüllung auf $t_p$ zwischen benachbarten Antennen (horizontal, vertikal, diagonal) zu messen, wie es für die Detektion systematischer Fehler zweckmäßig ist, wurde ferner ein Testaufbau verwendet. Um den Aufbau zu modellieren und eine realistische Näherung der Koppelsignale zu erhalten, wurde ein 4x 4 Antennenarray benutzt, das aus 16 zirkularen Bowtie-Antennen 10 besteht. Diese waren in vier Reihen und vier Spalten angeordnet. Das Prinzip der Anordnung der Antennen von Antenne 1 bis 16 kann auch der rein schematischen Figur 10 entnommen werden, wobei die Nummern 1 bis 16 jeweils in weißen Kästchen eingezeichnet sind, um die Antennenpositionen anzudeuten. In dieser Figur sind auch Zeit-Verzögerungen gezeigt, worauf im Folgenden noch eingegangen wird.

Die Größe einer einzelnen Bowtie-Antenne 10 von 3 cm x 6 cm (Mester at al. 2020) ist davon abgeleitet, dass gemäß dem vorstehend beschriebenen Ausführungsbeispiel einer erfindungsgemäßen Radar-Einrichtung 3000 Antennen 4 um das Lysimeter 3 positioniert sind. Die Mittelpunkte der Bowtie-Antennen 10 lagen 3 cm in horizontaler und 6 cm in vertikaler Richtung voneinander beabstandet. Die Wand des Lysimeters 3 wird durch eine 1 m x 1 m PVC Platte 11 mit einer Dicke von 0,03 m repräsentiert. Das Antennen-Array ist auf die Platte 11 mit Klebeband aufgeklebt. Eine zusätzliche Box aus PVC, die mit Wasser gefüllt werden kann, wurde hinter die PVC-Platte gestellt (in der Vorderansicht gemäß Figur 10 nicht erkennbar), um wechselnde Materialeigenschaften im Lysimeter 3 zu simulieren und damit den Einfluss der Lysimeterfüllung auf die Kopplungssignale untersuchen zu können. Ein Ricker-Wavelet (zweite Ableitung einer Gauß'schen Funktion) mit einer Amplitude von 1 V und einer Zentralfrequenz von $f_c$ = 750 MHz wurde als Sendesignal verwendet und mit einem Keysight Arbitrary Waveform Generator (AWG) M8190A 12 GSa/s erzeugt. Die Signale wurden mit 4 GSa/s abgetastet und um den Faktor 64 gemittelt, wobei ein Keysight DSAX91304A Infiniium Hochleistungsoszilloskop zum Einsatz kam. Ein koaxiales Kabel wurde dazu verwendet, den AWG mit dem Tx zu verbinden und eines, um den Rx mit dem Oszilloskop zu verbinden. Die Signale wurden unter Nutzung einer 250 Punkte Sinc Interpolierung interpoliert, um höhere Timing-Auflösungen zu erhalten, wenn Zeit-Verzögerungen zwischen Signalen über eine Kreuzkorrelation berechnet werden.

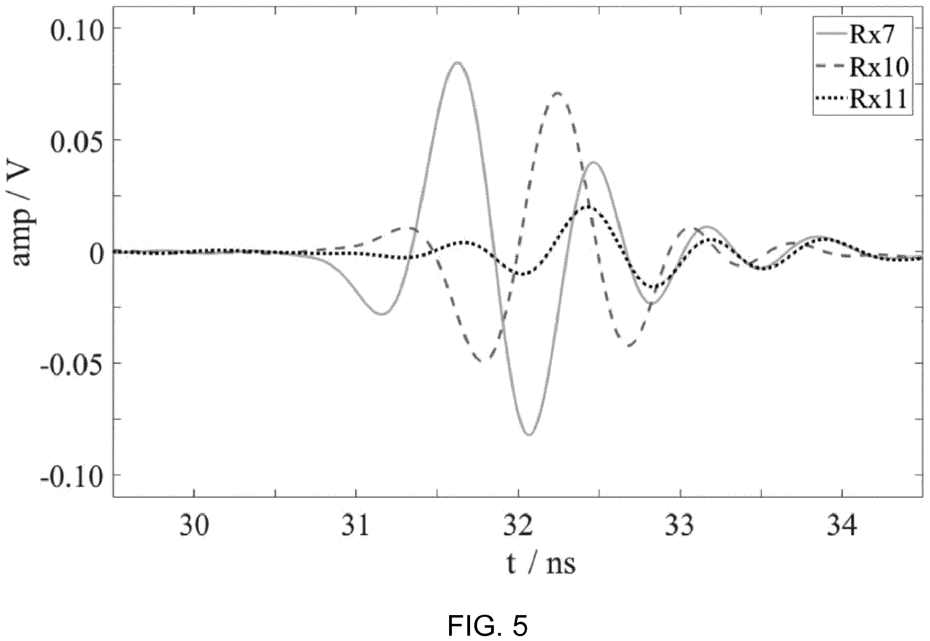

Die Figur 5 zeigt einen Graphen, in dem Koppelsignale für variierende Tx/Rx-Konfigurationen dargestellt sind. Auf der x-Achse ist dabei die Zeit in ns und auf der y-Achse die Amplitude in V aufgetragen. Die Figur veranschaulicht die Signalkopplung zwischen benachbarten Antennen 11 des Testaufbaus. Antenne Nr. 6 (vgl. Figur 10) ist in allen drei Fällen als Tx, also Sender, verwendet. Die Antennen 10 Nr. 7, 10 und 11 wurden als Rx verwendet (vgl. die Legende von Figur 5 mit Rx6, Rx10 und Rx11), um die horizontalen, vertikalen und diagonalen Koppelsignale aufzuzeigen. Alle drei Kopplungsrichtungen (horizontal, vertikal, diagonal) können prinzipiell für die Mesh-Kalibrierung genutzt werden und werden bezüglich ihrer Abhängigkeit von der Lysimeterfüllung analysiert.

Im Folgenden wird auf die Bestimmung der analogen Schaltkreis-Laufzeiten eingegangen.

Für eine erfolgreiche Kalibrierung wird die Summe der analogen Laufzeiten $t_{Tx} + t_{Rx}$ bestimmt (vgl. obige Gleichungen (3) und (7)). Gleichung (7) kann verwendet werden, um $t_{Tx} + t_{Rx}$ unter Durchführung von, z.B., reziproken WeitWinkel-Reflexions- und Brechungs-(WARR-)Messungen in Luft zwischen zwei Transceivern 2, zu bestimmen, ähnlich einer Korrektur des zeitlichen Nullpunktes, wie in (2) beschrieben. Der y-Achsenabschnitt der linearen Regression liefert dann den Wert für $t_{Tx} + t_{Rx}$.

Zu diesem Zweck können zwei Transceiver 2 in Luft platziert werden, um reziproke Messungen mit unterschiedlichen Transceiver-Abständen durchzuführen, bevor die Transceiver am Lysimeter 3 montiert werden. Es wurde der AWG und das Oszilloskop verwendet, um eine klassische Korrektur des zeitlichen Nullpunktes durchzuführen, um die Messgenauigkeit zu ermitteln, die mit einem idealen Messaufbau erreicht werden kann. Anschließend konnte die Messung von $t_{Tx} + t_{Rx}$ auf die gleiche Weise durchgeführt werden. Zu diesem Zweck wurde eine WARR-Messung in Luft zwischen Tx6 und einer einzelnen Bowtie-Antenne durchgeführt, wobei die einzelne Bowtie-Antenne sukzessive von der Antenne 6 (vgl. Figur 10), die in sendender Funktion auch als Tx6 bezeichnet werden kann, wegbewegt wurden. Für diese Messung wurde die PVC-Platte 11 entfernt und das Antennen-Array mit einer zusätzlichen Halterung (nicht in Figur 10 dargestellt) frei in der Luft angeordnet. Sowohl Tx6 als auch die einzelne Bowtie-Antenne wurden dabei in einer Höhe von ca. 0,55 m über dem Boden montiert und hatten einen Anfangsabstand von 0,15 m zueinander. Die einzelne Bowtie-Antenne wurde dann von Tx6 in Schritten von 0,05 m entfernt. Bei jedem Schritt wurde eine Messung mit einem Stacking von 256 durchgeführt.

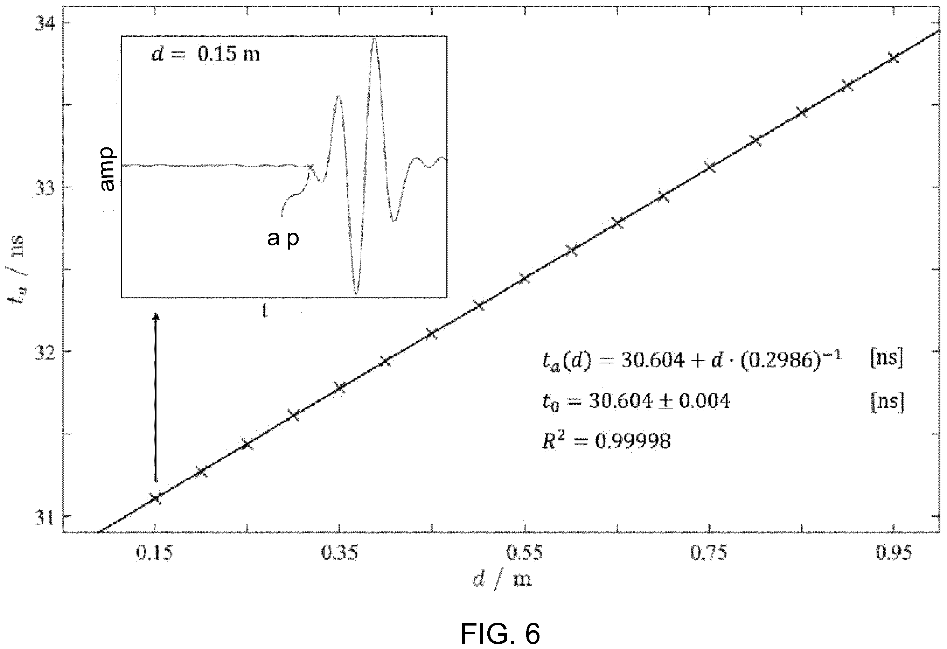

Die Ankunftszeiten, die für die Kalibrierung benötigt werden, werden wie folgt berechnet: erst wird die Ankunftszeit des Rohsignals mit dem kleinsten Abstand zu Tx6, also d = 0,15 m (siehe die innere Box in Figur 6), unter Nutzung eines Schwellwertes bestimmt. Der Schwellwert wurde auf 5y gesetzt, wobei $\gamma$ die Standardabweichung des Rauschens bei Abwesenheit eines eindeutigen Signals ist. Im zweiten Schritt wurde die Kreuzkorrelation des bei d = 0,15 m gemessenen Signals mit Signalen, die für größere Abstände d gemessen wurden, berechnet, um die Ankunftszeiten der korrespondierenden Signale zu berechnen (siehe die Kreuze in Figur 6). Die lineare Regression liefert $t_a$ (d) = 30,604 + d * 0,2986$^{-1}$. Das 99%-Konfidenzintervall des zeitlichen Nullpunktes ist 30,604 $\pm$ 0,004 ns. Der R$^2$-Wert nähert sich fast an 1 an. Dies zeigt eine hohe Genauigkeit der durchgeführten Korrektur des zeitlichen Nullpunktes über die WARR-Messung an. Darüber hinaus zeigt die berechnete Geschwindigkeit der Signale von v = 0,2986 m / ns eine Abweichung von 0,4 % zum erwarteten Wert von co = 0,2998 m / ns, was ein weiterer Hinweis auf eine erfolgreiche Korrektur des zeitlichen Nullpunktes ist. Zu beachten ist, dass die anfängliche Ankunftszeit des bei d = 0,15 gemessenen Signals auch anders gewählt werden kann. Dies würde zu einem neuen absoluten zeitlichen Nullpunkt führen, aber die Breite des Konfidenzintervalls würde gleich bleiben. Dieses Messverfahren gewährleistet eine hochgenaue Bestimmung der Summe der analogen Laufzeiten $t_{Tx} + t_{Rx}$.

Figur 6 zeigt einen Graphen zu Weitwinkel- und Brechungsmessungen in Luft, wobei Ankunftszeiten $t_a$ in Nanosekunden (y-Achse) für variierende Distanzen in Metern (x-Achse) angegeben sind. Eine lineare Regression liefert $t_0$ = 30,604 $\pm$ 0,004 ns. In der eingeschobenen Box, in der die Amplitude über der Zeit aufgetragen ist, ist ein einzelner, empfangener Puls bei einem Abstand von d = 0,15 m zwischen Tx6 und Rx die erste korrespondierende Ankunftszeit gezeigt.

Im Folgenden wird eine Simulation der Kalibrierung erörtert.

Die Standardabweichung der Mesh-Kalibrierung ist komplex analytisch zu analysieren und wurde deshalb unter Nutzung einer Simulation analysiert, welche die vorstehenden Überlegungen zu zufälligen Zeitfehlern berücksichtigt.

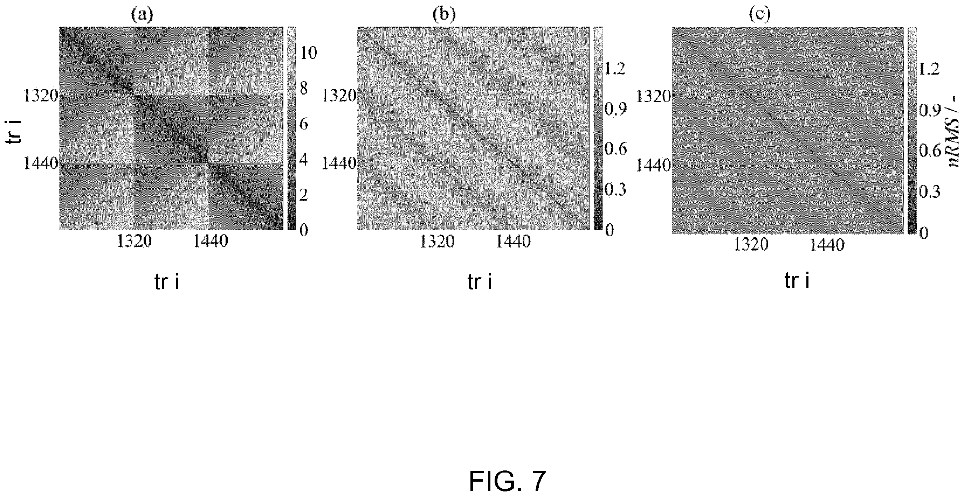

Figur 7 zeigt das nRMS für die drei Topologien aus Figur 4. In allen drei Fällen ist die Anzahl der Simulationsläufe N=10000. Die Teilfiguren zeigen konkret das nRMS für die Transceiver 1200 bis 1560. Es ist zu beachten, dass verschiedene Graustufen-Skalen verwendet werden.

Figur 7 zeigt konkret die nRMS-Werte gemäß Gleichung (21) für Mesh2 (Figur 7a), Mesh4 (Figur 7b) und Mesh8 (Figur 7c) im Bereich der Transceiver 1200 bis 1560. Die x- und y-Achse geben die Transceiver-Indizes tr i an. Die entsprechenden Transceiver 1200 bis 1560 sind in der Mitte des Lysimeters 3 bzw. Meshes angeordnet. Figur 7a zeigt ein klares Muster, das die nRMS-Werte in 120 x 120 Bereiche aufteilt. Jeder Bereich stellt eine Reihe um das Lysimeter 3 dar, wobei, wie schon beschrieben, in einer Reihe 120 Transceiver 2 um das Lysimeter 3 platziert sind. Der letzte Transceiver 2 pro Reihe führt eine reziproke Messung nur mit dem linken Nachbarn in derselben Reihe durch (siehe Figur 4a). Daraus folgt, dass zur Berechnung der Trigger-Offsets zwischen dem ersten und letzten Transceiver 2 pro Reihe 119 Zwischenmessungen erforderlich sind im Vergleich zu z.B. Mesh4 und Mesh8, wo eine zusätzliche Messung zwischen dem ersten und dem letzten Transceiver 2 pro Reihe durchgeführt wird. Dieses Muster wird in Figur 7a sichtbar, wo der Abstand zwischen dem ersten und letzten Transceiver 2 der Reihe 120 - 1 = 119 ist. Dies resultiert in größeren nRMS-Werten für Transceiver 2 mit größeren Abständen. Hier bezieht sich der Begriff Abstand auf die Anzahl der reziproken Messungen, die erforderlich sind, um zwei beliebige Transceiver 2 zu verbinden. Nach jeweils 120 Schritten sinkt der nRMS-Wert wieder durch eine direkte vertikale Messung zwischen den beiden ersten Transceivern 2 benachbarter Reihen.

Die Diagonale ist per Definition Null ($t_{i\,i}$ = 0). Außerdem sind die nRMS-Werte symmetrisch um die Diagonale, da per Definition $t_{ij} = -t_{j\,i}$. Der Gesamt-Mitteln RMS-Wert für Mesh2 ergibt 7,70.

Für Mesh4 und Mesh8 ist das für Mesh2 beobachtete Muster für den nRMS-Wert aufgrund weiterer Messungen zwischen zusätzlichen benachbarten Transceivern nicht sichtbar (vgl. Figur 7b und 7c). Der größte Abstand für zwei Transceiver in einer Reihe besteht nun zwischen Transceivern auf gegenüberliegenden Seiten des Lysimeters. Das Auffällige an den Figuren 7b und 7c ist, dass neben der Hauptdiagonalen weitere diagonale Linien sichtbar sind. Diese Linien resultieren aus weiteren direkten vertikalen Messungen, z.B. zwischen den Transceivern 2 und 122 oder Transceivern 3 und 123. Der Fehler zwischen den entsprechenden Trigger-Offsets wird dadurch reduziert. Die diagonalen Linien wiederholen sich alle 120 Transceiver 2, weil dies die Anzahl der Transceiver 2 pro Reihe ist. Für Mesh4 ist der mittlere *nRMS* gleich 1,22, was darauf hinweist, dass das größere System linearer Gleichungen den Fehler um einen Faktor von etwa 6 im Vergleich zu Mesh2 reduziert. Für Mesh8 beträgt der mittlere *nRMS* 0,93. Dieses Ergebnis zeigt, dass die Standardabweichung der Trigger-Offsets für beliebige Transceiver 2 kleiner sein kann, als die anfängliche Standardabweichung bei einer direkten reziproken Messung zwischen zwei Transceivern 2.

Zusätzlich zu dem *nRMS* wurde der mittlere *nRMS für* einen fixen Transceiver i als

$$\overline{nRMS_i} = \frac{1}{3000} \sum_{j=1}^{3000} nRMS_{i\ j}$$

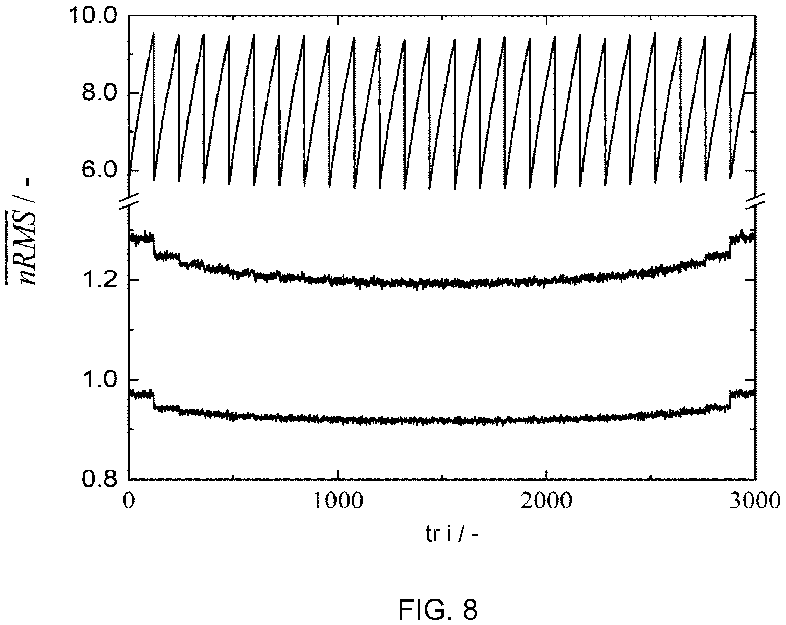

berechnet, um die Fehlerfortpflanzung weiter zu evaluieren (siehe Figur 8). Die Figur 8 zeigt den mittleren *nRMS* Wert $\overline{nRMS_i}$ für jede Spalte i von Figur 7 für Mesh2, Mesh4 und Mesh8. Für einen festen Transceiver i werden alle 3000 Trigger-Delays verwendet, um $\overline{nRMS_i}$ zu berechnen. Im Gegensatz zu Figur 7 sind in Figur 8 alle 3000 Transceiver-Indices tr i dargestellt.

Für Mesh2 ist ein Sägezahn-artiges Muster erkennbar. Dies ergibt sich aus der Anordnung der reziproken Messungen zwischen benachbarten Transceivern, denn der erste Transceiver pro Reihe hat einen geringeren Abstand zu Transceivern in anderen Reihen als der letzte Transceiver pro Reihe, wie bereits im Zusammenhang mit Figur 7 diskutiert. Bei Mesh4 und Mesh8 wird das Sägezahnmuster aufgrund zusätzlicher reziproker Messungen zwischen benachbarten Transceivern entfernt. Die $\overline{nRMS_i}$-Werte zeigen, dass die Transceiver in den oberen und unteren Reihen größere mittlere nRMS-Werte aufweisen. Dies, da die Transceiver in der obersten und untersten Reihe weniger reziproke Messungen durchführen, konkret drei/fünf anstelle von vier/acht, und dadurch einen Einfluss auf die folgenden Reihen haben. Daher tritt der signifikanteste Sprung der $\overline{nRMS_i}$-Werte nach Transceiver-Index 120 und vor Transceiver-Index 2880 für Mesh4 und Mesh8 auf. Dies sollte im Rahmen der FWI berücksichtigt werden, da diese Transceiver dazu neigen, weniger genaue zeitliche Nullpunkte zu haben, wenn die Mesh-Kalibrierung verwendet wird. Zu beachten ist, dass, wenn reziproke Messungen zwischen Transceivern aus der obersten und der untersten Reihe durchgeführt würden, die nRMSi-Werte nicht dieses stufenweise Muster zeigen würden.

Im Prinzip ist die Konstruktion des linearen Gleichungssystems wie in (12) nicht auf Messungen zwischen benachbarten Antennen beschränkt, solange die Reziprozität garantiert ist. Dann können die Signale durch den Boden zusammen mit Gleichung (8) verwendet werden, um das System linearer Gleichungen unter Verwendung einer im Vergleich zu Mesh8 noch größeren Matrix A zu lösen. Die Ergebnisse von Mesh8 sind aber bereits sehr gut und zeigen, dass die Standardabweichung mit weniger als $\sqrt{k\eta}$ für zunehmenden Abstand k zwischen Transceivern zunimmt, wie es der Fall für Mesh2 ist. Die Nutzung eines größeren linearen Gleichungssystems bietet daher signifikante Vorteile, was die Fehlerfortpflanzung angeht. Dieses Ergebnis besagt, dass zu erwartende Varianzen zufälliger Fehler im niedrigen Pikosekundenbereich für den Kalibrieransatz vernachlässigbar sind. Zu beachten ist, dass die Verwendung aller acht Nachbar-Transceiver eines Tx-Transceivers als zusätzliche *Rx* die Tomographie-Messzeit nicht signifikant erhöht, da die Kalibrierungsmessungen in die eigentliche Tomographie-Messung inkludiert werden können. Bei einer Tomographiemessung dient bevorzugt jeder Transceiver 2 einmal als Sender, während die verbleibenden Transceiver 2 als Empfänger dienen. Entsprechend finden reziproke Messungen zwischen allen Paaren von Transceivern 2 statt. Dann müssen lediglich die reziproken Messungen von Nachbarn gewählt werden, um die Kalibration durchzuführen.

Im Folgenden wird näher auf den Einfluss der Lysimeterfüllung eingegangen.

Gleiche Laufzeiten $t_p$ für Koppelsignale erlauben die Verifizierung und Prüfung der Annahme, dass $t_{Tx} + t_{Rx}$ für alle Transceiver gleich ist, wie vorstehend im Zusammenhang mit der Detektion systematischer Fehler erörtert. Deshalb wurden verschiedene Materialien hinter dem PVC 11 des Testaufbaus angeordnet und der Einfluss auf die Laufzeit wurde analysiert. Bevor der Einfluss des Materials auf die Kopplungssignale gemessen wurde, wurden reziproke Messungen zwischen den Antennen Nr. 6, 7, 10 und 11 durchgeführt (zu den Positionen der Antennen vgl. erneut Figur 10), um die allgemeinen Messunsicherheiten zu beurteilen. Zu diesem Zweck wurden die vier Antennen 10 des Testaufbaus durch Vertauschen der Kabel nacheinander als *Tx* und *Rx* verwendet, was in sechs reziproke Messungen resultierte. Die Zeit-Verzögerungen (englisch: time lags) für alle reziproken Messungen belaufen sich auf 4 ± 4 ps, was eine Unsicherheit von 4 ps ergibt, was in guter Übereinstimmung mit der vorstehend diskutierten, durch die Korrektur-Messungen des zeitlichen Nullpunktes erhaltenen Messgenauigkeiten ist. Zu beachten ist, dass die Zeit-Verzögerungen für das Messsystem aufgrund der unterschiedlichen Hardware unterschiedlich sein können. Jedoch wurde die Abweichung von ±4 ps als Referenz für eine erreichbare Messgenauigkeit genommen. Als nächstes wurde die Antenne Nr. 6 konstant als *Tx* und die restlichen 15 Antennen nacheinander als *Rx* verwendet. Für jede Tx/Rx-Kombination wurden zwei Messungen entweder mit Luft oder Wasser hinter dem PVC 11 durchgeführt. Dieses Experiment soll ein Worst-Case-Szenario für die Permittivität des Materials hinter dem PVC 11 bereitstellen ($\varepsilon_{Luft} \approx 1$, $\varepsilon_{Wasser} \approx 80$). Der Vergleich zwischen Luft und Wasser-Kopplung wurde über die Zeitverzögerungen zwischen den Signalen ausgewertet. Die Zeitverzögerungen wurden über die zeitliche Differenz zwischen den ersten lokalen Extrema beider Signale berechnet.

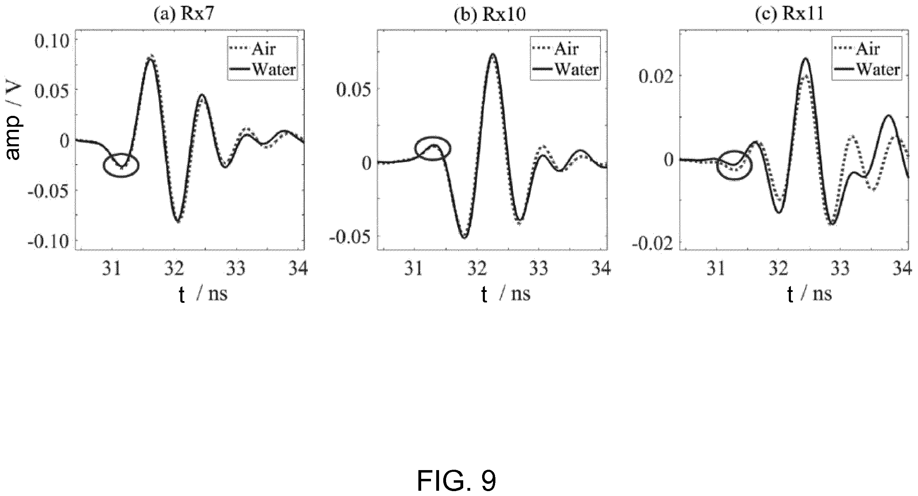

Figur 9 zeigt drei Graphen mit Koppelsignalen, wenn sich entweder Luft oder Wasser hinter dem PVC 11 befindet und zwar für Antenne Nr. 6 als *Tx* und (a) Antenne Nr. 7 als *Rx* (rechter Nachbar, vgl. Figur 10), (b) Antenne Nr. 10 als *Rx* (unterer Nachbar, vgl. Figur 10) bzw. (c) Antenne Nr. 11 als *Rx* (rechter unterer diagonaler Nachbar, vgl. Figur 10). Die roten Kreise markieren die ersten lokalen Extrema, die zur Berechnung der Zeit-Verzögerungen verwendet werden. Die Zeit-Verzögerungen für die drei Mess-Paare sind in Figur 10 gezeigt.

Figur 9 zeigt drei Beispiele von empfangenen Signalpaaren (Tx6 ist fest). Die Betrachtung der zugehörigen Signale in Figur 9 gibt Einblick in die Auswirkung einer Änderung der Permittivitäten hinter dem PVC 11 auf die Signale. Figur 9a zeigt, dass beim Vergleich der lokalen Minima und Maxima der beiden Signale für Rx7 die Zeitpunkte ähnlicher Peaks für das Luftsignal später eintreffen, was unerwartet ist, und sich für spätere Zeitpunkte tendenziell auseinander bewegen. Für Rx10 (Figur 9b) erscheinen die Peaks des Luftsignals früher als die Peaks des Wassersignals. Für Rx11 (Figur 9c) sind ausgeprägtere Zeitverzögerungen vor und nach dem maximalen Peak sichtbar. In diesem Fall kommt das Luft-Signal für Zeiten früher als der maximale Peak nach dem Wasser-Signal an und für den folgenden negativen Peak vor dem Wasser-Signal. Daraus folgt, dass ein wechselndes Material hinter dem PVC 11 einen Einfluss auf die Ausbreitung von Koppelsignalen hat. Der Einfluss variiert jedoch im Verlauf des Signals und ist kleiner für frühere Phasen für horizontale und vertikale Signale. Um dies weiter zu verdeutlichen, wurden die Differenzen der ersten lokalen Extrema-Zeiten für Luft- und Wasser-Signale in Pikosekunden für verschiedene Tx/Rx-Kombinationen berechnet. Die zugehörige Werte sind in Figur 10 in Pikosekunden angegeben. Die Zeitverzögerungen wurden über den zeitlichen Unterschied erster lokaler Extrema berechnet. Für die schraffierten Bereiche wurden keine Messungen durchgeführt.

In Figur 10 zeigen die Zeitverzögerungen einen geringeren Materialeinfluss für horizontale und vertikale Messungen. Dabei entspricht hell einer Zeitverzögerung kleiner gleich 10 ps, hellgrau einer Zeitverzögerung größer 10 und kleiner als 20 ps und dunkelgrau einer Zeitverzögerung von mehr als 20 ps. Der Einfluss von Wasser scheint systematisch zu sein. Die horizontalen Messungen zeigen eine Zeitverzögerung zwischen Luft und Wasser-Messungen von bis zu 10 ps. Es kann getestet werden, ob die Position der Empfänger (Rx5 befindet sich am Rande des Arrays) einen Einfluss auf die Signale hat, z. B. durch die Verwendung eines größeren Antennen-Arrays. Bei vertikalen Messungen liegen die Zeitverzögerungen bereits unter der ermittelten Messgenauigkeit von $\pm 4$ ps und machen daher vertikale Koppelsignale gut geeignet, um die Laufzeiten analoger Schaltungen zu testen. Diagonale Koppelsignale sind weniger geeignet für die Analyse, da sie die stärkste Abhängigkeit von der Lysimeterfüllung zeigen.

Der Vergleich von Luft- und Wasser-Signalen nach dem Testaufbau stellt ein Worst-Case-Szenario hinsichtlich der Material-Permittivitäten dar. Bei Bodenmessungen variieren die Permittivitäten in einem Bereich von $\varepsilon_r = 5 - 40$. Daher ist es vertretbar anzunehmen, dass der Vergleich früherer Signalzeiten und realistischere Materialvariationen hinter dem PVC den Einfluss der Lysimeterfüllung auf die Kopplungssignale noch mehr reduzieren. Dann können die horizontalen Kopplungssignale zusätzlich zu vertikalen Kopplungssignalen zum Testen der Laufzeiten der Analogschaltungen verwendet werden. Es folgt, dass die Annahme, dass $f_{Tx} + t_{Rx}$ für alle Transceiver gleich ist, getestet werden kann, da der Einfluss der Lysimeterfüllung auf die Koppelsignale vernachlässigbar ist.

Referenzen

Annan, A. P. (2003). "Ground Penetrating Radar: Principles, Procedures & Applications". In: Sensors & Software Incorporated, p. 278. doi: 10.1190/1.9781560801719.ch11.

Binley, A. et al. (Nov. 2001). "High-resolution characterization of vadose zone dynamics using cross-borehole radar". In: Water Resources Research 37.11, pp. 2639-2652. doi: 10.1029/2000WR000089.

Bolten, J. D. et al. (Mar. 1, 2010). "Evaluating the Utility of Remotely Sensed SoilMoisture Retrievals for Operational AgriculturalDrought Monitoring". In: IEEE Journal of selected topics in applied earth observations and remote sensing 3.1.

Cassidy, N. J. (2009). "Ground Penetrating Radar Data Processing, Modelling and Analysis". In: Ground Penetrating Radar Theory and Applications. Elsevier, pp. 141-176. doi: 10.1016/B978-0-444-53348-7.00005-3.

Catapano, I. et al. (2014). "Oil spill monitoring via microwave tomography enhanced GPR surveys". In: Journal of Applied Geophysics 108, pp. 95-103. Ciampoli, L. B. et al. (2019). "Railway Ballast Monitoring by GPR: A Test-Site Investigation". In: MDPI remote sensing.

De Benedetto, D. et al. (2019). "Mapping an Agricultural Field Experiment by Electromagnetic Induction and Ground Penetrating Radar to Improve Soil Water Content Estimation". In: agronomy.

Diamanti, N. et al. (2018). "The WARR Machine: System Design, Implementation and Data". In: Journal of Environmental and Engineering Geophysics 24 (4). doi: 10.2113/JEEG23. 4.469.

Dinh, K. et al. (Mar. 2019). "Automated visualization of concrete bridge deck condition from GPR data". In: NDT & E International 102, pp. 120-128. doi: 10.1016/j.ndteint.2018.11.015.

Dobson, M. et al. (Jan. 1985). "Microwave Dielectric Behavior of Wet Soil-Part II: Dielectric Mixing Models". In: IEEE Transactions on Geoscience and Remote Sensing GE-23.1, pp. 35-46. doi: 10.1109/TGRS.1985.289498. Doolittle, J. A. et al. (July 2014). "The use of electromagnetic induction techniques in soil studies". In: Geoderma 223-225, pp. 33-45. doi: 10.1016/j.geoderma.2014.01.027.

Ernst, J. R. et al. (Sept. 2007). "Full-Waveform Inversion of Crosshole Radar Data Based on 2-D Finite-Difference Time-Domain Solutions of Maxwell's Equations". In: IEEE Transactions on Geoscience and Remote Sensing 45.9, pp. 2807-2828. doi: 10.1109/TGRS.2007.901048.

Gerhards, H. et al. (July 2008). "Continuous and simultaneous measurement of reflector depth and average soil-water content with multichannel groundpenetrating radar". In: GEOPHYSICS 73.4, J15-J23. doi: 10.1190/1.2943669.

Gueting, N. et al. (Jan. 2017). "High resolution aquifer characterization using crosshole GPR full-waveform tomography: Comparison with direct-push and tracer test data". In: Water Resources Research 53.1, pp. 49-72. doi: 10.1002/2016WR019498.

Hammond, D. S. et al. (Mar. 2011). "Parameterizing road construction in routebased road weather models: can ground-penetrating radar provide any answers?" In: Measurement Science and Technology 22.5, p. 057001. doi: 10.1088/0957-0233/22/5/057001.

Hannes, M. et al. (Aug. 2015). "A comprehensive filtering scheme for high-resolution estimation of the water balance components from high-precision lysimeters". In: Hydrology and Earth System Sciences 19.8, pp. 3405-3418. doi: 10.5194/hess- 19-3405-2015.

Hugenschmidt, J. et al. (2006). "GPR inspection of concrete bridges". In: Cement & Concrete Composites 28, pp. 384-392.

Huisman, J. A. et al. (2003). "Measuring Soil Water Content with Ground Penetrating Radar: A Review". In: Vadose Zone Journal 2, pp. 476-491.

Hyun, S.-Y. et al. (July 2007). "The laboratory scaled-down model of a groundpenetrating radar for leak detection of water pipes". In: Measurement Science and Technology 18.9, pp. 2791-2799. doi: 10.1088/0957-0233/18/9/008.

Ihamouten, A. et al. (2018). "Full-waveform inversion using a stepped-frequency GPR to characterize the tack coat in hot-mix asphalt (HMA) layers of flexible pavements". In: NDT and E International 95, pp. 17-25.

Jol, H. M. (Dec. 11, 2008). Ground Penetrating Radar: Theory and Applications. Ed. by H. Jol. ELSEVIER Science. isbn: 9780444533487.

Kaufmann, M. S. et al (Jan. 2020). "Simultaneous multichannel multi-offset groundpenetrating radar measurements for soil characterization". In: Vadose Zone Journal 19.1. doi: 10.1002/vzj2.20017.

Klotzsche, A., F. Jonard, et al. (2018). "Measuring Soil Water Content with Ground Penetrating Radar: A Decade of Progress". In: Vadose Zone Journal 17.1. doi: 10.2136/vzj2018.03.0052.

Klotzsche, A., J. van der Kruk, et al. (Aug. 2014). "Detection of spatially limited highporosity layers using crosshole GPR signal analysis and full-waveform inversion". In: Water Resources Research 50.8, pp. 6966-6985. doi: 10.1002/2013wr015177.

Klotzsche, A., H. Vereecken, et al. (Nov. 2019). "Review of crosshole groundpenetrating radar full-waveform inversion of experimental data: Recent developments, challenges, and pitfalls". In: GEOPHYSICS 84.6, H13-H28. doi: 10.1190/geo2018-0597.1.

Lameri, S. et al. (Aug. 2017). "Landmine detection from GPR data using convolutional neural networks". In: 2017 25th European Signal Processing Conference (EUSIPCO). IEEE. doi: 10.23919/EUSIPCO.2017.8081259.

Liu, X. et al. (Dec. 2017). "Measurement of soil water content using groundpenetrating radar: a review of current methods". In: International Journal of Digital Earth 12.1, pp. 95-118. doi: 10.1080/17538947.2017.1412520.

Meles, G. A. et al. (Sept. 2010). "A New Vector Waveform Inversion Algorithm for Simultaneous Updating of Conductivity and Permittivity Parameters From Combination Crosshole/Borehole-to-Surface GPR Data". In: IEEE Transactions on Geoscience and Remote Sensing 48.9, pp. 3391-3407. doi: 10.1109/TGRS.2010.2046670.

Mester, A. et al. (Mar. 25, 2020). "Small broadband antennas for multi-channel GPR monitoring system". In.

Oberröhrmann, M. et al. (Sept. 2012). "Optimization of acquisition setup for cross-hole: GPR full-waveform inversion using checkerboard analysis". In: Near Surface Geophysics 11.2, pp. 197-209. doi: 10.3997/1873-0604.2012045.

Olhoeft, G. R. (Mar. 2000). "Maximizing the information return from ground penetrating radar". In: Journal of Applied Geophysics 43.2-4, pp. 175-187. doi: 10.1016/s0926-9851(99)00057-9.

Paz, C. et al. (2017). "Current uses of ground penetrating radar in groundwater-dependent ecosystems research". In: Science of the Total Environment.

Pütz, T. et al. (Sept. 2016). "TERENO-SOILCan: a lysimeter-network in Germany observing soil processes and plant diversity influenced by climate change". In: Environmental Earth Sciences 75.18. doi: 10.1007/s12665-016-6031-5.

Qin, H. et al. (Jan. 2021). "Analysis of Forward Model, Data Type, and Prior Information in Probabilistic Inversion of Crosshole GPR Data". In: Remote Sensing 13.2, p. 215. doi: 10.3390/rs13020215.

Radzevicius, S. (July 2015). "Least-squares curve fitting for velocity and time zero". In: 2015 8th International Workshop on Advanced Ground Penetrating Radar (IWAGPR). IEEE. doi: 10.1109/IWAGPR.2015.7292677.

Roth, K. et al. (Oct. 1990). "Calibration of time domain reflectometry for water content measurement using a composite dielectric approach". In: Water Resources Research 26.10, pp. 2267-2273. doi: 10.1029/WR026i010p02267.

Saarenketo, T. et al. (2000). "Road evaluation with ground penetrating radar". In: Journal of Applied Geophysics 43, pp. 119-138.

Sai, B. et al. (Feb. 2, 2004). "GPR Phase-Based Techniques for Profiling Rough Surfaces and Detecting Small, Low-Contrast Landmines Under Flat Ground". In: IEEE Transactions on Geosciense and Remote Sensing.

Samouëlian, A. et al. (Sept. 2005). "Electrical resistivity survey in soil science: a review". In: Soil and Tillage Research 83.2, pp. 173-193. doi: 10.1016/j.still.2004.10.004.

Schmalholz, J. et al. (Nov. 2004). "Imaging of Water Content Distributions inside a Lysimeter using GPR Tomography". In: Vadose Zone Journal 3.4, pp. 1106-1115. doi: 10.2136/vzj2004.1106.

Slob, E. et al. (Sept. 2010). "Surface and borehole ground-penetrating-radar developments". In: GEOPHYSICS 75.5, 75A103-75A120. doi: 10.1190/1.3480619.

Stoffregen, H. et al. (Oct. 2002). "Accuracy of soil water content measurements using ground penetrating radar: comparison of ground penetrating radar and lysimeter data". In: Journal of Hydrology 267.3-4, pp. 201-206. doi: 10.1016/s0022-1694(02)00150-6.

Topp, G. C. et al. (1980). "Electromagnetic Determination of Soil and Water Content: Measurements in Coaxial Transmission Lines". In: WATER RESOURCES RESEARCH 16.3, pp. 574-582.

Vereecken, H. et al. (2016). "Modeling Soil Processes: Review, Key Challenges, and New Perspectives". In: Vadose Zone Journal.

Viriyametanont, K. et al. (Apr. 2008). "Radar survey of concrete elements: Effect of concrete properties on propagation velocity and time zero". In: NDT & E International 41.3, pp. 198-207. doi: 10.1016/j.ndteint.2007.10.001.

Wijewardana, Y. N. S. et al. (2017). "Ground-penetrating radar (GPR) responses for subsurface salt contamination and solid waste: modeling and controlled lysimeter studies". In: Environment Monitoring Assess.

Wu, K. et al. (2019). "A new drone-borne GPR for soil moisture mapping". In: Remote Sensing of Environment.

Xu, B. et al. (Aug. 1993). "On the completeness of data sets with multielectrode systems for electrical resistivity survey". In: Geophysical Prospecting 41.6, pp. 791-801. doi: 10.1111/j.1365-2478.1993.tb00885.x.

Xu, X. et al. (May 2014). "Measuring soil layer thickness in land rearrangement with GPR data". In: Measurement Science and Technology 25.7, p. 075802. doi: 10.1088/0957-0233/25/7/075802.

Yang, M. et al. (2021). "Impact of planting time soil moisture on cereal crop yield in the Upper Blue Nile Basin A novel insight towards agricultural water management". In: Agricultural Water Management.

Yelf, R. (2004). "Where is True Time Zero?" In: Proceedings of the 10th International Conference on Ground Penetrating Radar IEEE.

Zhou, Z. et al. (Nov. 2020). "3D aquifer characterization of the Hermalle-sous-Argenteau test site using crosshole ground-penetrating radar amplitude analysis and full-waveform inversion". In: GEOPHYSICS 85.6, H133-H148. doi: 10.1190/geo2020-0067.1.

**Patentansprüche**

1. Verfahren zur zerstörungsfreien Untersuchung einer Probe (7) mit einer Radar-, insbesondere Geo-Radar-Einrichtung (1), wobei die Radar-Einrichtung (1) mehrere Transceiver (2), die jeweils sowohl als Sender als auch als Empfänger für elektromagnetische Messsignale fungieren können, umfasst, bei dem

    - eine oder mehrere reziproke Messungen zwischen Transceiver-Paaren durchgeführt werden (Schritt S1), wobei eine reziproke Messung jeweils eine Hin- und eine Rück-Messung einschließt, wobei für die Hin-Messung der eine Transceiver (2) des oder des jeweiligen Paares als Sender und der andere Transceiver (2) als Empfänger fungiert, und für die Rück-Messung der andere Transceiver (2) des oder des jeweiligen Paares als Sender und der eine Transceiver (2) als Empfänger fungiert,
    - unter Rückgriff auf die oder die jeweilige Ankunftszeit des Messsignals an dem einen Transceiver (2), insbesondere einer Antenne (4) dieses, während der oder der jeweiligen Hin-Messung, Hin-Ankunftszeit ($t_{aij}$), und der Ankunftszeit des Messsignals an dem anderen Transceiver, insbesondere einer Antenne dieses, während der oder der jeweiligen Rück-Messung, Rück-Ankunftszeit ($t_{aji}$), wenigstens eine Laufzeit ($t_p$, $t_{p\,ij}$) des Messsignales zwischen den beiden Transceiver (2) ermittelt wird (Schritt S2), und
    - aus der wenigstens einen Laufzeit ($t_p$, $t_{pij}$, $t_{pji}$) auf Eigenschaften der zu untersuchenden Probe (7), insbesondere eine Permittivität dieser, geschlossen wird (Schritt S3).

2. Verfahren nach Anspruch 1, **dadurch gekennzeichnet, dass** jeder Transceiver (2) einen Sende-Schaltkreis (5) und einen Empfangs-Schaltkreis (6) und bevorzugt eine Antenne (4) umfasst, bevorzugt, wobei die Laufzeit ($t_p$) zwischen einem Transceiver $i$ (2) und einem Transceiver $j$ (2) eines Transceiver-Paares bestimmt wird mittels der Formel

$$t_p = \frac{t_{a\,i\,j} + t_{a\,j\,i}}{2} - (t_{\mathrm{Tx}} + t_{\mathrm{Rx}}),$$

wobei $t_{a\,i\,j}$ die Hin-Ankunftszeit, $t_{a\,j\,i}$ die Rück-Ankunftszeit, $t_{\mathrm{Tx}}$ die analoge Sendepfad-Laufzeit und $t_{\mathrm{Rx}}$ die analoge Empfangspfad-Laufzeit des jeweiligen Transceivers (2) ist, insbesondere, wobei davon ausgegangen wird, dass die Summe von $t_{\mathrm{Tx}}$ und $t_{\mathrm{Rx}}$ bekannt ist.

3. Verfahren nach Anspruch 2, **dadurch gekennzeichnet, dass** die Laufzeit $t_{p\,ij}$ zwischen einem Transceiver i (2) und einem Transceiver j (2) eines Transceiver-Paares bestimmt wird mittels der Formel

$$t_{p\,i\,j} = t_{a\,i\,j} - t_{i\,j} - (t_{Tx} + t_{Rx}),$$

wobei $t_{a\,ij}$ die Hin-Ankunftszeit, $t_{Tx}$ die analoge Laufzeit des Sende-Schaltkreises und $t_{Rx}$ die analoge Laufzeit Empfangs-Schaltkreises des jeweiligen Transceivers (2) ist, insbesondere, wobei davon ausgegangen wird, dass die Summe von $t_{Tx}$ und $t_{Rx}$ bekannt ist, und wobei $t_{ij}$ das Trigger-Offset der Transceiver i und j (2) eines Transceiver-Paares ist, das definiert ist als

$$t_{i\,j} := t_i - t_j$$

mit der Trigger-Zeit $t_i$ des Transceivers $i$ und der Trigger-Zeit $t_j$ des Transceivers j, bevorzugt, wobei Trigger-Offsets berechnet werden mittels der Formel

$$t_{i\,j} = \frac{t_{a\,i\,j} - t_{a\,j\,i}}{2}.$$

4. Verfahren nach einem der vorhergehenden Ansprüche, **dadurch gekennzeichnet, dass** die Trigger-Offsets für Paare von Transceivern (2), die keine unmittelbaren Nachbarn sind, aus den Trigger-Offsets von bevorzugt dazwischenliegenden jeweils in vertikaler und/oder horizontaler und/oder diagonaler Richtung direkt benachbarten Transceiver-Paaren berechnet werden.

5. Verfahren nach Anspruch 3 und 4, **dadurch gekennzeichnet, dass** Trigger-Offsets $t_{ij}$ von Transceiver-Paaren als Input für die Formel $t_{p\,ij} = t_{a\,ij}t_{ij} - (t_{Tx} + t_{Rx})$ unter Verwendung zweier Matrizen ermittelt werden, bevorzugt, wobei eine Matrix A definiert wird, so dass

$$A\ t = t_{measured}$$

gilt, wobei $t$ die Trigger-Zeiten mehrerer, insbesondere aller Transceiver (2) umfasst, und $t_{measured}$ ein Vektor ist, der die Trigger-Offsets von Transceiver-Paaren, bevorzugt solchen, die in vertikaler und/oder horizontaler und/oder diagonaler Richtung direkt benachbart sind, umfasst, die über $t_{i\,j} = \frac{t_{a\,i\,j} - t_{a\,j\,i}}{2}$ ermittelt werden, und

$$B\ t = t_{searched}$$

gilt, wobei die weitere Matrix $B$ gesuchte Transceiver-Kombinationen umfasst, und der Vektor $t_{searched}$ gesuchte Trigger-Offsets enthält, und

$$t_{searched} = B\ A^+ t_{measured}$$

gilt, wobei $A^+$ die Pseudoinverse der Matrix A ist, die bevorzugt durch QR-Faktorisierung oder Singulärwert-Zerlegung berechnet wird.

6. Verfahren nach einem der vorhergehenden Ansprüche, **dadurch gekennzeichnet, dass** die Transceiver (2) in einer oder mehreren Reihen und/oder einer oder mehreren Spalten angeordnet sind, insbesondere, wobei reziproke Messungen für Transceiver-Paare durchgeführt werden, die in horizontaler Richtung direkt zueinander benachbart sind, und/oder, dass reziproke Messungen für Transceiver-Paare durchgeführt werden, die in vertikaler Richtung direkt zueinander benachbart sind, und/oder, dass reziproke Messungen für Transceiver-Paare durchgeführt werden, die in diagonaler Richtung direkt zueinander benachbart sind.

7. Verfahren nach einem der vorhergehenden Ansprüche, **dadurch gekennzeichnet, dass** die Transceiver (2) an wenigstens einem insbesondere hohlzylinderförmigen Basiskörper (3) und/oder zumindest im Wesentlichen äquidistant voneinander beabstandet angeordnet sind, und/oder dass alle Transceiver (2) baugleich ausgebildet sind, und/oder dass jeder Transceiver (2) einmal als Sender fungiert, während der oder alle anderen Transceiver (2) als Empfänger fungieren, und/oder dass für alle möglichen Paare von Transceivern (2) jeweils eine reziproke Messung mit einer Hin- und einer Rück-Messung durchgeführt wird.

8. Verfahren nach einem der vorhergehenden Ansprüche, **dadurch gekennzeichnet, dass** aus der Laufzeit ($t_p$, $t_{pij}$, $t_{pji}$) auf die Permittivität und/oder Leitfähigkeit der zu untersuchenden Probe (7) geschlossen wird, bevorzugt, wobei mittels einer Volle-Wellenform-Inversion auf die räumliche Verteilung der Permittivität und/oder Leitfähigkeit der zu untersuchenden Probe (7) geschlossen wird.

9. Verfahren nach einem der vorhergehenden Ansprüche, **dadurch gekennzeichnet, dass** es sich bei der zu untersuchenden Probe um eine Bodenprobe (7) handelt.

10. Verfahren nach Anspruch 8 und Anspruch 9, **dadurch gekennzeichnet, dass** aus der Permittivität der Wassergehalt der Bodenprobe (7) bestimmt wird.

11. Verfahren nach einem der vorhergehenden Ansprüche, **dadurch gekennzeichnet, dass** die Probe über einen vorgegebenen Messzeitraum mehrfach untersucht wird.

12. Radar-, insbesondere Geo-Radar-Einrichtung (1), zur Durchführung des Verfahrens nach einem der vorhergehenden Ansprüche, umfassend mehrere, insbesondere 3000, Transceiver (2), die jeweils sowohl als Sender als auch als Empfänger für elektromagnetische Messsignale fungieren können und bevorzugt jeweils einen Sende-Schaltkreis (5) und einen Empfangs-Schaltkreis (6) und eine Antenne (4) aufweisen, und ein Steuer- und Auswerte-Modul, das zur Durchführung des Verfahrens nach einem der vorhergehenden Ansprüche ausgebildet und eingerichtet ist.

13. Radar-Einrichtung nach Anspruch 12, **dadurch gekennzeichnet, dass** wenigstens ein insbesondere hohlzylinderförmiger Basiskörper (3), bevorzugt ein Lysimeter, vorgesehen ist, an dem die Transceiver (2) bevorzugt außenseitig und/oder in einer oder mehreren Reihen und/oder einer oder mehreren Spalten und/oder zumindest im Wesentlichen äquidistant voneinander beabstandet gehalten sind, und/oder dass eine zentrales, der Triggerung der Transceiver dienendes Trigger-Modul (8) vorgesehen ist, bevorzugt, wobei das Trigger-Modul (8) mit jedem Transceiver (2) über wenigstens ein Kabel direkt oder indirekt verbunden ist.

14. Computerprogramm, umfassend Befehle, die bei der Ausführung des Programms durch einen Computer diesen veranlassen, die Schritte des Verfahrens nach einem der Ansprüche 1 bis 11 auszuführen.

15. Computerprogrammprodukt, umfassend Befehle, die bei der Ausführung des Programms durch einen Computer diesen veranlassen, die Schritte des Verfahrens nach einem der Ansprüche 1 bis 11 auszuführen.

FIG. 1

EP 4 113 155 A1

FIG. 2

FIG. 3

FIG. 4

FIG. 5

$t_a(d) = 30.604 + d \cdot (0.2986)^{-1}$ [ns]

$t_0 = 30.604 \pm 0.004$ [ns]

$R^2 = 0.99998$

FIG. 6

FIG. 7

EP 4 113 155 A1

FIG. 8

(a) Rx7      (b) Rx10      (c) Rx11

FIG. 9

EP 4 113 155 A1

FIG. 10

FIG. 11

Europäisches Patentamt

European Patent Office

Office européen des brevets

**EUROPÄISCHER RECHERCHENBERICHT**

**Nummer der Anmeldung**

EP 22 17 3971

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | MALAJNER MARKO ET AL: "Soil type characterization for moisture estimation using machine learning and UWB-Time of Flight measurements", MEASUREMENT, INSTITUTE OF MEASUREMENT AND CONTROL. LONDON, GB, Bd. 146, 29. Juni 2019 (2019-06-29), Seiten 537-543, XP085761150, ISSN: 0263-2241, DOI: 10.1016/J.MEASUREMENT.2019.06.042 [gefunden am 2019-06-29] | 1,2,6-15 | INV. G01S7/02 G01S7/40 G01S13/00 G01S13/87 G01S13/88 G01N22/04 G01N33/24 G01S7/41 G01V3/12 |
| A | * Abbildungen 1, 3-5, 10-12 * * Seite 538, linke Spalte, Zeilen 12-13 * * Seite 538, linke Spalte, Zeile 53 – Seite 538, rechte Spalte, Zeile 2 * * Seite 538, rechte Spalte, Zeilen 40-42 * * Seite 539, linke Spalte, Zeilen 1-4, 13-16, 32 * * Seite 539, linke Spalte, Zeile 41 – Seite 539, rechte Spalte, Zeile 18 * * Seite 540, rechte Spalte, Zeile 36 * * Seite 541, linke Spalte, Zeile 19 – Seite 541, rechte Spalte, Zeile 6 * * Seite 541, rechte Spalte, Zeilen 26-49 * ----- -/-- | 3-5 | |

**RECHERCHIERTE SACHGEBIETE (IPC)**

G01S
G01N
G01V

Der vorliegende Recherchenbericht wurde für alle Patentansprüche erstellt

| Recherchenort | Abschlußdatum der Recherche | Prüfer |
|---|---|---|
| München | 12. Oktober 2022 | Rodríguez González |

EPO FORM 1503 03.82 (P04C03)

Seite 1 von 2

Europäisches Patentamt
European Patent Office
Office européen des brevets

# EUROPÄISCHER RECHERCHENBERICHT

Nummer der Anmeldung

EP 22 17 3971

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | GALAGEDARA L W ET AL: "An analysis of the ground-penetrating radar direct ground wave method for soil water content measurement", HYDROLOGICAL PROCESSING, WILEY, CHICHESTER, GB, Bd. 17, Nr. 18, 15. Dezember 2003 (2003-12-15), Seiten 3615-3628, XP071715045, ISSN: 0885-6087, DOI: 10.1002/HYP.1351 * Zusammenfassung; Abbildung 5 * * Seite 3616, Zeile 29 – Seite 3617, Zeile 2 * * Seite 3619, Zeilen 4-14, 35-43 * * Seite 3621, Zeilen 15-18 * * Seite 3623, Zeilen 5-11 * * Seite 3627, Zeilen 9-13 * ----- | 1-15 | |
| A | GALAGEDARA L W ET AL: "Field studies of the GPR ground wave method for estimating soil water content during irrigation and drainage", JOURNAL OF HYDROLOGY, ELSEVIER, AMSTERDAM, NL, Bd. 301, Nr. 1-4, 20. Januar 2005 (2005-01-20), Seiten 182-197, XP027852282, ISSN: 0022-1694 [gefunden am 2005-01-20] * Seite 194, rechte Spalte, Zeile 2 – Seite 195, rechte Spalte, Zeile 9 * ----- | 1-15 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 12. Oktober 2022 | Rodríguez González |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................................
& : Mitglied der gleichen Patentfamilie, übereinstimmendes Dokument

EPO FORM 1503 03.82 (P04C03)

Seite 2 von 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **ANNAN, A. P.** Ground Penetrating Radar: Principles, Procedures & Applications. *Sensors & Software Incorporated,* 2003, 278
- **BINLEY, A. et al.** High-resolution characterization of vadose zone dynamics using cross-borehole radar. *Water Resources Research,* November 2001, vol. 37 (11), 2639-2652
- **BOLTEN, J. D. et al.** Evaluating the Utility of Remotely Sensed SoilMoisture Retrievals for Operational Agricultural Drought Monitoring. *IEEE Journal of selected topics in applied earth observations and remote sensing,* 01. Marz 2010, vol. 3 (1), 1
- Ground Penetrating Radar Data Processing, Modelling and Analysis. **CASSIDY, N. J.** Ground Penetrating Radar Theory and Applications. Elsevier, 2009, 141-176
- **CATAPANO, I. et al.** Oil spill monitoring via microwave tomography enhanced GPR surveys. *Journal of Applied Geophysics,* 2014, vol. 108, 95-103
- **CIAMPOLI, L. B. et al.** Railway Ballast Monitoring by GPR: A Test-Site Investigation. *MDPI remote sensing.,* 2019
- **DE BENEDETTO, D et al.** Mapping an Agricultural Field Experiment by Electromagnetic Induction and Ground Penetrating Radar to Improve Soil Water Content Estimation. *agronomy,* 2019
- **DIAMANTI, N. et al.** The WARR Machine: System Design, Implementation and Data. *Journal of Environmental and Engineering Geophysics,* 2018, vol. 24 (4
- **DINH, K.** Automated visualization of concrete bridge deck condition from GPR data. *NDT & E International,* Marz 2019, vol. 102, 120-128
- **DOBSON, M. et al.** Microwave Dielectric Behavior of Wet Soil-Part II: Dielectric Mixing Models. *IEEE Transactions on Geoscience and Remote Sensing GE-23.1,* Januar 1985, 35-46
- **DOOLITTLE, J. A. et al.** The use of electromagnetic induction techniques in soil studies. *Geoderma,* Juli 2014, vol. 223-225, 33-45
- **ERNST, J. R. et al.** Full-Waveform Inversion of Crosshole Radar Data Based on 2-D Finite-Difference Time-Domain Solutions of Maxwell's Equations. *IEEE Transactions on Geoscience and Remote Sensing,* September 2007, vol. 45.9, 2807-2828

- **GERHARDS, H. et al.** Continuous and simultaneous measurement of reflector depth and average soil-water content with multichannel groundpenetrating radar. *GEOPHYSICS,* Juli 2008, vol. 73.4, J15-J23
- **GUETING, N. et al.** High resolution aquifer characterization using crosshole GPR full-waveform tomography: Comparison with direct-push and tracer test data. *Water Resources Research,* Januar 2017, vol. 53.1, 49-72
- **HAMMOND, D. S. et al.** Parameterizing road construction in routebased road weather models: can ground-penetrating radar provide any answers?. *Measurement Science and Technology,* Marz 2011, vol. 22.5, 057001
- **HANNES, M. et al.** A comprehensive filtering scheme for high-resolution estimation of the water balance components from high-precision lysimeters. *Hydrology and Earth System Sciences,* August 2015, vol. 19.8, 3405-3418
- **HUGENSCHMIDT, J. et al.** GPR inspection of concrete bridges. *Cement & Concrete Composites,* 2006, vol. 28, 384-392
- **HUISMAN, J. A. et al.** Measuring Soil Water Content with Ground Penetrating Radar: A Review. *Vadose Zone Journal,* 2003, vol. 2, 476-491
- **HYUN, S.-Y. et al.** The laboratory scaled-down model of a groundpenetrating radar for leak detection of water pipes. *Measurement Science and Technology,* Juli 2007, vol. 18.9, 2791-2799
- **IHAMOUTEN, A. et al.** Full-waveform inversion using a stepped-frequency GPR to characterize the tack coat in hot-mix asphalt (HMA) layers of flexible pavements. *NDT and E International,* 2018, vol. 95, 17-25
- **JOL, H. M.** Ground Penetrating Radar: Theory and Applications. ELSEVIER Science, 11. Dezember 2008
- **KAUFMANN, M. S. et al.** Simultaneous multichannel multi-offset groundpenetrating radar measurements for soil characterization. *Vadose Zone Journal,* Januar 2020, vol. 19, 1
- **KLOTZSCHE, A. ; F. JONARD et al.** Measuring Soil Water Content with Ground Penetrating Radar: A Decade of Progress. *Vadose Zone Journal,* 2018, vol. 17, 1

- **KLOTZSCHE, A. ; J. VAN DER KRUK et al.** Detection of spatially limited highporosity layers using crosshole GPR signal analysis and full-waveform inversion. *Water Resources Research,* August 2014, vol. 50 (8), 6966-6985
- **KLOTZSCHE, A. ; H. VEREECKEN et al.** Review of crosshole groundpenetrating radar full-waveform inversion of experimental data: Recent developments, challenges, and pitfalls. *GEOPHYSICS,* November 2019, vol. 84 (6), H13-H28
- Landmine detection from GPR data using convolutional neural networks. **LAMERI, S. et al.** 2017 25th European Signal Processing Conference (EUSIPCO). IEEE, August 2017
- **LIU, X. et al.** Measurement of soil water content using groundpenetrating radar: a review of current methods. *International Journal of Digital Earth,* Dezember 2017, vol. 12 (1), 95-118
- **MELES, G. A. et al.** A New Vector Waveform Inversion Algorithm for Simultaneous Updating of Conductivity and Permittivity Parameters From Combination Crosshole/Borehole-to-Surface GPR Data. *In: IEEE Transactions on Geoscience and Remote Sensing,* September 2010, vol. 48.9, 3391-3407
- **MESTER, A. et al.** *Small broadband antennas for multi-channel GPR monitoring system,* 25. Marz 2020
- **OBERRÖHRMANN, M. et al.** Optimization of acquisition setup for cross-hole: GPR full-waveform inversion using checkerboard analysis. *Near Surface Geophysics,* September 2012, vol. 11 (2), 197-209
- **OLHOEFT, G. R.** Maximizing the information return from ground penetrating radar. *Journal of Applied Geophysics,* Marz 2000, vol. 43.2-4, 175-187
- **PAZ, C. et al.** Current uses of ground penetrating radar in groundwater-dependent ecosystems research. *Science of the Total Environment,* 2017
- **PÜTZ, T. et al.** TERENO-SOILCan: a lysimeter-network in Germany observing soil processes and plant diversity influenced by climate change. *Environmental Earth Sciences,* September 2016, vol. 75 (18
- **QIN, H. et al.** Analysis of Forward Model, Data Type, and Prior Information in Probabilistic Inversion of Crosshole GPR Data. *Remote Sensing,* Januar 2021, vol. 13 (2), 215
- Least-squares curve fitting for velocity and time zero. **RADZEVICIUS, S.** 2015 8th International Workshop on Advanced Ground Penetrating Radar (IWAGPR). IEEE, Juli 2015
- **ROTH, K. et al.** Calibration of time domain reflectometry for water content measurement using a composite dielectric approach. *Water Resources Research,* Oktober 1990, vol. 26 (10), 2267-2273
- **SAARENKETO, T. et al.** Road evaluation with ground penetrating radar. *Journal of Applied Geophysics,* 2000, vol. 43, 119-138
- **SAI, B. et al.** GPR Phase-Based Techniques for Profiling Rough Surfaces and Detecting Small, Low-Contrast Landmines Under Flat Ground. *IEEE Transactions on Geosciense and Remote Sensing,* 02. Februar 2004
- **SAMOUËLIAN, A. et al.** Electrical resistivity survey in soil science: a review. *Soil and Tillage Research,* September 2005, vol. 83 (2), 173-193
- **SCHMALHOLZ, J. et al.** Imaging of Water Content Distributions inside a Lysimeter using GPR Tomography. *Vadose Zone Journal,* November 2004, vol. 3 (4), 1106-1115
- **SLOB, E. et al.** Surface and borehole ground-penetrating-radar developments. *GEOPHYSICS,* September 2010, vol. 75 (5), 75A103-75A120
- **STOFFREGEN, H. et al.** Accuracy of soil water content measurements using ground penetrating radar: comparison of ground penetrating radar and lysimeter data. *Journal of Hydrology,* Oktober 2002, vol. 267.3-4, 201-206
- **TOPP, G. C. et al.** Electromagnetic Determination of Soil and Water Content: Measurements in Coaxial Transmission Lines. *WATER RESOURCES RESEARCH,* 1980, vol. 16. (3), 574-582
- **VEREECKEN, H. et al.** Modeling Soil Processes: Review, Key Challenges, and New Perspectives. *Vadose Zone Journal,* 2016
- **VIRIYAMETANONT, K. et al.** Radar survey of concrete elements: Effect of concrete properties on propagation velocity and time zero. *NDT & E International,* April 2008, vol. 41 (3), 198-207
- **WIJEWARDANA, Y. N. S. et al.** Ground-penetrating radar (GPR) responses for subsurface salt contamination and solid waste: modeling and controlled lysimeter studies. *Environment Monitoring Assess,* 2017
- **WU, K. et al.** A new drone-borne GPR for soil moisture mapping. *Remote Sensing of Environment,* 2019
- **XU, B. et al.** On the completeness of data sets with multielectrode systems for electrical resistivity survey. *Geophysical Prospecting,* August 1993, vol. 41 (6), 791-801
- **XU, X. et al.** Measuring soil layer thickness in land rearrangement with GPR data. *Measurement Science and Technology,* Mai 2014, vol. 25 (7), 075802
- **YANG, M. et al.** Impact of planting time soil moisture on cereal crop yield in the Upper Blue Nile Basin A novel insight towards agricultural water management. *Agricultural Water Management,* 2021
- **YELF, R.** Where is True Time Zero?. *Proceedings of the 10th International Conference on Ground Penetrating Radar IEEE,* 2004

- **ZHOU, Z. et al.** 3D aquifer characterization of the Hermalle-sous-Argenteau test site using crosshole ground-penetrating radar amplitude analysis and full-waveform inversion. *GEOPHYSICS,* November 2020, vol. 85 (6), H133-H148